# EUROPEAN PATENT SPECIFICATION

(11) **EP 2 353 593 B1**
(45) Date of publication and mention of the grant of the patent: **22.08.2012**
(21) Application number: 11160994.7
(22) Date of filing: 22.08.2003
(51) Int. Cl.: A61K 31/13, A01N 33/02, A61P 31/18

(54) **Non-nucleoside reverse transcriptase inhibitors**
Nicht-nukleosidische Reverse Transkriptase Inhibitoren
Inhibiteurs non-nucleosidiques de la transkriptase inverse

(30) Priority: 23.08.2002 WO PCT/US02/26816
(43) Date of publication of application: 10.08.2011
(62) Divisional of application: 03786506.0
(73) Proprietor: Ardea Biosciences, Inc., San Diego, CA 92121 (US)
(72) Inventor: Girardet, Jean-Luc, Aliso Viejo, CA 92656 (US); Zhang, Zhijun, Harbor City, CA 90710 (US); Hamatake, Robert, Aliso Viejo, CA 92656 (US); De la Rosa, Martha A., Fountain Valley, CA 92708 (US); Gunic, Esmir, Irvine, CA 92614 (US); Hong, Zhi, Aliso Viejo, CA 92656 (US); Kim, HongWoo, Irvine, CA 92606 (US); Koh, Yung-hyo, Irvine, CA 92620 (US); Nilar, Shahul, Irvine, CA 92606 (US); Shaw, Stephanie, Rowland Heights, CA 91748 (US); Yao, Nanhua, Irvine, CA 92606 (US)
(74) Representative: Viering, Jentschura & Partner

(56) References cited:
- EP-A2- 0 303 301
- JP-A- 63 185 965
- MEENAKSHI SHRIMALI, K. S. DIXIT, J. P. BARTHWAL: J. IND. CHEM. SOC, vol. 68, no. 8, 1991, pages 466-469, XP009111543,
- MARTHA DE LA ROSA, HONG WOO KIM, ESMIR GUNIC, CHERYL JENKET, UYEN BOYLE ET AL.: "Tri-substituted triazoles as potent non-nucleoside inhibitors of the HIV-1 reverse transkriptase", BIOORGANIC & MEDICINAL CHEMISTRY LETTERS, vol. 16, 2006, pages 4444-4449, XP002512986,

## Description

### Field of The Invention

The field of the invention is enzyme inhibition, and particularly *in vitro* and *in vivo* inhibition of reverse transcriptases.

### Background of The Invention

Numerous treatments for HIV are known in the art, and among other pharmaceutically active compounds, reverse transcriptase inhibitors have provided significant therapeutic effect to many HIV infected patients. For example, Lamivudine (3TC) or Zidovudine (AZT) are relatively well tolerated antiretroviral drugs. However, numerous viral strains have recently emerged with marked resistance against these compounds. To overcome resistance to at least some degree, new nucleoside-type inhibitors may be administered (alone or in combination with other nucleoside-type inhibitors), and exemplary alternative drugs include Stavudine (d4T), Didanosine (ddI), Combivir (a combination of Lamivudine and Zidovudine), and Trizivir (a combination of 3TC, AZT, and Abacavir).

Unfortunately, development of resistance against one nucleoside-type inhibitor may also be accompanied by resistance (to at least some degree) against another nucleoside-type inhibitor, frequently necessitating a switch to a different class of pharmaceutically active molecules. In such cases, a patient may receive a protease inhibitor (*e.g*., sequinavir, indinavir, nelfinavir, etc.), typically in combination with other anti retroviral agents. However, the relatively complex administration regimen of such combinations often proves an organizational and financial challenge to many patients, and compliance is frequently less than desirable.

In a somewhat better tolerated combination therapy, nucleoside-type inhibitors may be combined with non-nucleoside-type inhibitors. Non-nucleoside-type inhibitors (*e.g*., Nevirapine, Delavirdine, Efavirenz) are a structurally relatively inhomogeneous group of compounds and are thought to bind in a non-nucleoside pocket of the reverse transcriptase, thereby significantly increasing antiviral efficacy where nucleoside-type inhibitors is employed. While use of non-nucleoside-type inhibitors seems to provide a promising new class of antiviral drugs, several disadvantages still remain. For example, the cost for currently known non-nucleoside-type inhibitors is relatively high, and a single mutation in the viral reverse transcriptase can induce a cross resistance against a wide class of non-nucleoside reverse transcriptase inhibitors. Moreover, there is only a limited number of non-nucleoside-type inhibitors available for treatment of an HIV infected patient.

Shrimali et al. (J. Ind. Chem. Soc (1991) 68, 8, 466-469) disclose the evaluation of substituted indolyl-1,2,4-triazoles for anticonvulsant and analgesic activity. US 5,939,462 discloses α-alkoxy and α-thioalkoxyamide compositions as NPY5 receptor antagonists.

Thus, although various compositions and methods for inhibition of reverse transcriptase, and especially a reverse transcriptase from HIV are known in the art, all or almost all of them have one or more disadvantages. Moreover, the HIV virus has a relatively high frequency of mutation, which often leads to drug resistance to current treatments. Therefore, there is still a need to provide new compositions and methods for inhibition of reverse transcriptases.

### Summary of the Invention

The present invention is directed to compounds, uses and compositions for inhibition of a reverse transcriptase wherein various carbonyl amide compounds act as inhibitory compounds of a reverse transcriptase.

In one aspect, the inventive subject matter relates to a compound having the structure HET-L-C(Y)NR₁R₂, wherein HET comprises a 5 or 6 member ring heterocycle, L is a linker in which at least two atoms form a contiguous chain, wherein one of the two atoms is covalently bound to H, and wherein another one of the two atoms is covalently bound to the carbonyl atom, Y is oxygen, sulfur, or NH, R₁ is selected from the group consisting of hydrogen, halogen, and methyl, or R₁ forms a ring with R₂ via a chain of between 1-5 atoms; and R₂ is selected from the group consisting of a substituted or unsubstituted aryl, a cycloalkanyl, a cycloalkenyl, and a substituted or unsubstituted heterocycle.

In particularly preferred embodiments, HET is a substituted triazole or imidazole, and it is even more preferred that the substituted triazole or imidazole is substituted with a first substituent (*e.g*., methyl) and a second substituent (*e.g*., toluyl), wherein at least one of the first and second substituents includes an aryl group. Moreover, it is generally preferred that L is -X₁-CR₃R₄-, wherein X₁ is selected from the group consisting of S, O, S(O), S(O)₂, NH, NR₃ and CR₃R₄; and wherein R₃ and R₄ are independently hydrogen, halogen, lower alkyl, lower cycloalkyl, lower alkenyl, lower alkynyl, NH₂, OH, and SH. In still further preferred aspects, L is selected from the group consisting of -S-CH₂-, -S(O)-CH₂-, -S(O)₂-CH₂-, -O-CH₂-, NHCH₂, N(Me)CH₂ and -CH₂-CH₂-, and/or Y is O. In still further preferred compounds, R₁ is hydrogen and R₂ is a substituted aryl or heteroaryl, and more preferably R₂ comprises an ortho-substituted phenyl in which the substituent is a halogen or methyl.

Especially contemplated uses include those in which the reverse transcriptase is an HIV reverse transcriptase, and most preferably in which the HIV reverse transcriptase is resistant to a non-nucleoside analog reverse transcriptase inhibitor. Contemplated uses may be for applications *in vivo* and/or *in vitro,* and may further include a step in which a compound is converted to a prodrug, and/or a step in which the reverse transcriptase is presented with a second inhibitor (*e.g*., non-nucleoside reverse transcriptase inhibitor and a nucleoside reverse transcriptase inhibitor).

Therefore, it is contemplated that a compound is used in the manufacture of a medicament for treating an HIV infected patient where a pharmaceutical composition comprising a compound according to Structure I is administered to a patient at a dosage effective to reduce viral propagation, wherein Structure I is HET-L-C(Y)NR₁R₂, and wherein HET comprises a heterocycle, L is a linker in which at least two atoms form a contiguous chain, wherein one of the two atoms is covalently bound to HET, and wherein another one of the two atoms is covalently bound to the carbonyl atom, Y is oxygen, sulfur, or NH, R₁ is selected from the group consisting of hydrogen, halogen, and methyl, or R₁ forms a ring with R₂ via a chain of between 1-5 atoms, and R₂ is selected from the group consisting of a substituted or unsubstituted aryl, a cycloalkanyl, a cycloalkenyl, and a substituted or unsubstituted heterocycle. With respect to particularly preferred substituents, the same considerations as described above apply.

Consequently, it is contemplated that a pharmaceutical composition will include a compound of the structure HET-L-C(Y)NR₁R₂ (with substituents as described above) wherein the compound is present in a concentration effective to inhibit a reverse transcriptase in a cell of a patient when administered to the patient.

In still further contemplated aspects of the inventive subject matter, a compound has a general structure of HET-W-C(R₁)(R₂)-C(Y)-N(R₄R₅), wherein HET comprises a nitrogen-containing substituted heterocycle, W is O, S(O), S(O)₂, NH, NR₁ or CH₂, R₁ and R₂ are independently hydrogen, lower alkyl, lower cycloalkyl, lower alkenyl, lower alkynyl, halogen, OH, SH, NH₂, N₃, O-alkyl, or CH₂OH Y is O, S, or NR₃, wherein R₃ is hydrogen, lower alkyl, lower alkenyl, lower alkynyl, or hydroxy, O-alkyl, or CH₂OH R₄ is hydrogen, lower alkyl, lower alkenyl, or lower alkynyl, or R₄ forms a ring with R₅ via a chain of between 1-5 atoms, and R₅ is selected from the group consisting of a substituted or unsubstituted aryl, a cycloalkanyl, a cycloalkenyl, and a substituted or unsubstituted heterocycle.

In yet another aspect of the inventive subject matter, a compound has a general structure of HET-S-C(R₁)(R₂)-C(Y)-N(R₄R₅), wherein HET comprises a nitrogen-containing substituted heterocycle, R₁ and R₂ are independently hydrogen, lower alkyl, lower cycloalkyl, lower alkenyl, lower alkynyl, halogen, OH, SH, NH₂, N₃, O-alkyl, or CH₂OH, and with the proviso that R₁ and R₂ are not hydrogen at the same time, Y is O, S, or NR₃, wherein R₃ is hydrogen, lower alkyl, lower alkenyl, lower alkynyl, or hydroxy, O-alkyl, or CH₂OH R₄ is hydrogen, lower alkyl, lower alkenyl, or lower alkynyl, or R₄ forms a ring with R₅ via a chain of between 1-5 atoms, and R₅ is selected from the group consisting of a substituted or unsubstituted aryl, a cycloalkanyl, a cycloalkenyl, and a substituted or unsubstituted heterocycle.

In still other aspects of the inventive subject matter, a compound has a general structure of HET-W-C(R₁)(R₂)-C(Y)-N(R₄R₅), wherein HET comprises a nitrogen-containing substituted heterocycle other than a triazole, W is O, S, S(O), S(O)₂, NH, N(Me) or CH₂, R₁ and R₂ are independently hydrogen, lower alkyl, lower alkenyl, lower alkynyl, halogen, OH, SH, NH₂, N₃, O-alkyl, or CH₂OH, Y is O, S, or NR₃, wherein R₃ is hydrogen, lower alkyl, lower alkenyl, lower alkynyl, or hydroxy, O-alkyl, or CH₂OH R₄ is hydrogen, lower alkyl, lower alkenyl, or lower alkynyl, or R₄ forms a ring with R₅ via a chain of between 1-5 atoms, and R₅ is selected from the group consisting of a substituted or unsubstituted aryl, a cycloalkanyl, a cycloalkenyl, and a substituted or unsubstituted heterocycle.

Various objects, features, aspects and advantages of the present invention will become more apparent from the following detailed description of preferred embodiments of the invention, along with the accompanying drawings in which like numerals represent like components.

### Detailed Description

The inventors surprisingly discovered that a reverse transcriptase, and particularly the reverse transcriptase of HIV may be inhibited by numerous compounds that include a carbonyl amide moiety. Consequently, uses and compositions are contemplated that inhibit a reverse transcriptase *in vitro* and *in vivo.* Further especially contemplated uses are for treatment of a patient infected with HIV, and particularly contemplated compositions include selected carbonyl amide compounds and pharmacological compositions thereof.

As used herein, the term "halogen" refers to a fluorine, bromine, chlorine, or iodine, which is typically covalently bound to another atom (e.g., carbon). As further used herein, the term "hydroxyl" refers to a -OH group. As still further used herein, the term "carbonyl atom" refers to a carbon atom to which three atoms are covalently bound, wherein one of the three atoms is bound to the carbon atom via a double bond (which may be partially delocalized). Thus, particularly contemplated carbonyl atoms include carbon atoms in a carboxamide group, a carboxamidine group, and a thiocarboxamide group.

The term "alkyl" as used herein refers to a cyclic, branched, or straight hydrocarbon in which all of the carbon-carbon bonds are single bonds, and the term "lower alkyl" refers to a cyclic, branched, or straight chain alkyl of one to ten carbon atoms (*e.g*., methyl, ethyl, n-propyl, i-propyl, n-butyl, t-butyl, i-butyl (or 2-methylpropyl), cyclopropylmethyl, i-amyl, n-amyl, hexyl, etc.). The term "cycloalkyl" as used herein refers to a cyclic or polycyclic alkyl group containing 3 to 15 carbons. For polycyclic groups, these may be multiple condensed rings in which one of the distal rings may be aromatic (*e.g*., indanyl, tetrahydronaphthalene, etc.).

Similarly, the term "alkenyl," as used herein refers to an alkyl in which at least one carbon-carbon bond is a double bond. Thus, the term "lower alkenyl" includes all alkenyls with one to ten carbon atoms. The term "cycloalkenyl" as used herein refers to a cyclic or polycyclic group containing 3 to 15 carbons and at least one double bond. Likewise, the term "alkynyl," as used herein refers to an alkyl or alkenyl in which at least one carbon-carbon bond is a triple bond. Thus, the term "lower alkynyl" includes all alkynyls with one to ten carbon atoms.

As still further used herein, the term "alkoxy" refers to a -OR group, wherein R is lower alkyl, substituted lower alkyl, acyl, aryl, substituted aryl, arylalkyl, substituted arylalkyl, heteroarylalkyl, cycloalkyl, substituted cycloalkyl, cycloheteroalkyl, or substituted cycloheteroalkyl. Similarly, the term "aryloxy" refers to a -OAr group, wherein Ar is an aryl, substituted aryl, heteroaryl, or substituted heteroaryl group.

Furthermore, the terms "aryl" and "Ar" are used interchangeably herein and refer to an aromatic carbocyclic group having at least one aromatic ring (*e.g*., phenyl or biphenyl) or multiple condensed rings in which at least one ring is aromatic, (*e.g*., 1,2,3,4-tetrahydronaphthyl, naphthyl, anthryl, or phenanthryl). Similarly, the terms "heterocycle" or "heterocyclic ring" are used interchangeably herein and refer to a saturated, partially or entirely unsaturated, or aromatic carbocyclic group having a single ring (*e.g*., morpholino, pyridyl or furyl) or multiple condensed rings (*e.g*., naphthpyridyl, quinoxalyl, quinolinyl, or indolizinyl) which include at least one heteroatom within the ring(s). The term "heteroatom" as used herein refers to an atom other than carbon (*e.g*., S, O, or N), which can optionally be substituted with, e.g., hydrogen, halogen, lower alkyl, alkoxy, lower alkylthio, trifluoromethyl, amino, amido, carboxyl, hydroxyl, aryl, aryloxy, heterocycle, heteroaryl, substituted heteroaryl, nitro, cyano, alkylthio, thiol, sulfamido and the like. Thus, the term "heteroaryl" refers to a heterocycle in which at least one heterocyclic ring is aromatic.

Still further, the term "substituted" as used herein means that a hydrogen atom that is covalently bound to a group or atom (or a free electron pair or electron pair of a double bond of an atom) is replaced by a covalently bound non-hydrogen substituent, including hydroxyl, thiol, alkylthiol, halogen, alkoxy, amino, amido, nitro, carboxyl, cycloalkyl, heterocycle, cycloheteroalkyl, acyl, carboxyl, aryl, aryloxy, heteroaryl, arylalkyl, heteroarylalkyl, alkyl, alkenyl, alknyl, and cyano.

The term "prodrug" as used herein refers to a modification of contemplated compounds, wherein the modified compound exhibits less pharmacological activity (as compared to the unmodified compound) and wherein the modified compound is converted within a target cell (*e.g*., T-cell) or target organ (*e.g*., lymph node) back into the unmodified form. For example, conversion of contemplated compounds intro prodrugs may be useful where the active drug is too toxic for safe systemic administration, or where the contemplated compound is poorly absorbed by the digestive tract, or where the body breaks down the contemplated compound before reaching its target.

As further used herein, the term "inhibiting a reverse transcriptase" refers to a reduction of the formation of DNA from a template RNA or DNA by a reverse transcriptase, wherein the reduction may be directly or indirectly achieved in various manners. For example, direct inhibition includes suicide, competitive and non-competitive inhibition, allosteric inhibition, or binding of an inhibitor in a non-nucleoside pocket. Examples on indirect inhibition include depletion of nucleosides for DNA synthesis, induction or contribution to conformational changes, etc.

As still further used herein, the term "reducing [or: to reduce] viral propagation" means that the titer of a virus in a sample is lowered, wherein the reduction may include various manners, including partial or total inhibition of viral replication, partial or total inhibition of viral protein processing or assembly, viral entry into or exit from an infected cell, and/or clearance of the virus from a system via an immune response to the virus.

### Contemplated Compounds

The inventors generally contemplate that all compounds of Formula (I) are suitable for use herein:

HET-L-C(Y)NR₁R₂ (I)

wherein HET comprises a substituted or unsubstituted heterocycle, which may or may not be aromatic; L is a linker in which at least two atoms form a contiguous chain, wherein one of the two atoms is covalently bound to the heterocycle, and wherein another one of the two atoms is covalently bound to the carbonyl carbon atom; Y is O, S, or NR₃; R₁ and R₃ are independently selected from the group consisting of hydrogen, halogen, and optionally substituted alkyl, alkenyl, or alkynyl (preferably lower alkyl); and R₂ is selected from the group consisting of a substituted or unsubstituted aryl, a cycloalkyl, a cycloalkenyl, and a substituted or unsubstituted heterocycle (which may include one or more double bonds, and which may further be aromatic).

With respect to the heterocycle it is preferred that at least one, and more typically at least two of the heteroatoms are nitrogen, and that the two heteroatoms are connected to each other in the heterocycle via a covalent bond. Consequently, particularly suitable heterocycles include a triazole (most preferably a 1,2,4-triazole) or imidazole ring system. In alternative aspects, however, suitable heterocycles may also include 5-, and 6-membered rings with at least one heteroatom (*e.g*., O, N, or S), wherein such rings may further be coupled or fused to at least one other ring (which may or may not include a heteroatom).

Particularly preferred heterocycles further include at least one, and even more preferably at least two substituents, wherein suitable substituents independently include a substituted and/or an unsubstituted alkyl, a substituted and/or an unsubstituted alkyl, a substituted and/or an unsubstituted alkynyl, a substituted and/or an unsubstituted alkynyl, wherein each of the two substituents may further include one or more heteroatoms. However, in even more preferred aspects, contemplated heterocycles will include a lower alkyl (and most preferably a methyl, a halogen atom or trifluoromethyl) as one substituent and a substituted or unsubstituted phenyl (*e.g*., halogenated or toluyl) or a substituted or unsubstituted quinoline as the other substituent.

Consequently, particularly preferred heterocycles will have a structure according to Formula (II) 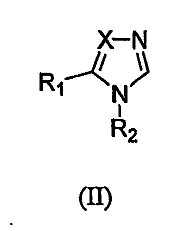 wherein R₁ and R₂ are independently hydrogen, halogen, lower alkyl, cycloalkyl, alkenyl, alkynyl, aryl, heteroaryl, alkaryl (all of which may be substituted), OH, SH, NO₂, NR₁R₂ (with R₁ and R₂ as defined immediately above), CF₃, N₃, and/or an O-alkyl, and X is N or CR₁ (with R₁ as defined immediately above).

In further contemplated aspects, it should be recognized that the structure and chemical nature of suitable linkers may vary substantially. For example, where it is desired that the linker has a relatively rigid character (*e.g*., at least one, and more typically two degrees of rotational freedom are restricted), suitable linkers may include a double and/or triple bond, or include one or more atoms in a planar configuration (*e.g*., aromatic, conjugated, or carbonyl structure). On the other hand, where it is desirable that the linker has flexibility to at least some degree, suitable linkers may include an alkyl group, or an oxygen or sulfur atom. Thus, suitable linkers may also include various heteroatoms, and particularly preferred heteroatoms are oxygen and sulfur (in various oxidation states).

Consequently, contemplated linkers include particularly those in which at least two atoms form a contiguous chain (via a covalent bond), wherein one of the two atoms is covalently bound to the heterocycle (preferably to a carbon atom of HET), and wherein another one of the two atoms is covalently bound to the carbonyl carbon atom of contemplated compounds. Thus, particularly preferred linkers will have a structure according to Formula (III)

-X₁-CR₃R₄- (III)

wherein X₁ is a heteroatom, and most preferably S, S(O), S(O)₂, O, or NR₅ wherein R₅ is preferably hydrogen, or substituted or unsubstituted alkyl (most preferably lower alkyl). Alternatively, X₁ may also include a carbon atom and may thus have the structure - (CR₅R₆)ₙ- wherein n is between one and five, and wherein R₃, R₄, R₅, and R₆ are independently hydrogen, halogen, lower alkyl, lower alkenyl, lower alkynyl, NH₂, OH, and/or SH. Therefore, suitable linkers will include those having the structure -S-CH₂-, -S(O)-CH₂-, -S(O)₂-CH₂-, -O-CH₂-, -NHCH₂, -N(CH₃)CH₂, and -CH₂-CH₂-.

Moreover, it should be appreciated that the carbonyl carbon atom of Formula (I) may be covalently bound to various atoms/groups Y, and particularly suitable groups Y include those in which Y is O (to form a carboxamide), S (to form a thiocarboxamide), and NR (to form a carboxamidine), wherein R may be hydrogen, or a substituted or unsubstituted lower alkyl. Suitable alternative R include all those that will. (form with N, or) provide a hydrogen bond donor or acceptor group. Consequently, Y of Formula (I) may be O, S, or NR, with R as defined above, especially including hydrogen, lower alkyl, lower alkenyl, lower alkynyl, or hydroxy, O-alkyl, or CH₂OH.

Similarly, and with further respect to compounds according to Formula (I), the nature of the substituents R₁ and R₂ of the nitrogen atom that is covalently bound to the carbonyl carbon may vary considerably, and all known substituents of secondary amines are contemplated herein. Therefore, R₁ and R₂ in Formula (I) may be independently hydrogen, substituted or unsubstituted alkyl, substituted or unsubstituted alkenyl, substituted or unsubstituted alkynyl, substituted or unsubstituted aryl, all of which may further include one or more heteroatoms. However, it is generally preferred that one of R₁ and R₂ is relatively small (*e.g*., hydrogen, methyl, trifluoromethyl, etc.), while the other of R₁ and R₂ comprises an aryl group. Especially preferred aryl groups will be substituted, most preferably in ortho-position, and may further include a substituent in para-position (*e.g*., ortho-substituted phenyl with halogen or methyl as substituent). Therefore, especially contemplated R₁ will include a hydrogen and lower alkyl (which may be further substituted [*e.g*., trifuoromethyl]), while especially contemplated R₂ include an aryl, a cycloalkyl, a cycloalkenyl, a heteroaryl, and a heterocycle.

In one especially preferred aspect, the heterocycle is covalently bound to the linker via a group other than -S- or -O-, and the linker has a relatively short and relatively flexible structure of -W-C(R₁)(R₂)-. Consequently, contemplated compounds will have a structure according to Formula (IV)

HET-W-C(R₁)(R₂)-C(Y)-N(R₄R₅) (IV)

wherein HET is defined as in Formula (I) above, and wherein C(Y)-N(R₄R₅) is defined as C(Y)-N(R₁R₂) in Formula (I) above. With respect to W, it is generally contemplated that all groups and/or atoms other than -S- and -O- are appropriate, and particularly preferred groups include S(O), S(O)₂, NH, NR₁ and CH₂. Particularly preferred R₁ and R₂ and relatively small radicals, and it is especially preferred that R₁ and R₂ are independently lower alkyl, lower alkenyl, lower alkynyl (all of which may be further substituted), hydrogen, halogen, OH, SH, NH₂, N₃, O-alkyl, or CH₂OH.

Alternatively, where it is desired that the heterocycle is covalently bound to the linker via a -S-, -O-, or other group, and where the linker is relatively short and flexible, contemplated compounds may have a structure according to Formula (V)

HET-W-C(R₁)(R₂)-C(Y)-N(R₄R₅) (V)

in which HET, R₁, R₂, R₄ and R₅ are defined as in Formula (IV) above, and in which W and Y are defined as Formula (I) above with the exception that Y is not O.

In a still further contemplated aspect, particularly preferred compounds include those in which the heterocyclic base is a disubstituted 1,2,4-triazole or a disubstituted imidazole, and suitable compounds may have a structure according to Formula (VI)

HET-W-C(R₁)(R₂)-C(Y)-N(R₄R₅) (VI)

wherein HET comprises a disubstituted 1,2,4-triazole or a disubstituted imidazole, wherein at least one substituents of HET is a substituted aryl, and wherein the substituted aryl is covalently bound to a nitrogen of HET; wherein W is O, S, S(O), S(O)₂, NH, NR₁ or CH₂, wherein Y is defined as in Formula (I) above, and wherein R₁, R₂, R₄, and R₅ are independently as defined above in Formula (IV). Thus, particularly preferred compounds will have a structure according to Formulae A or B 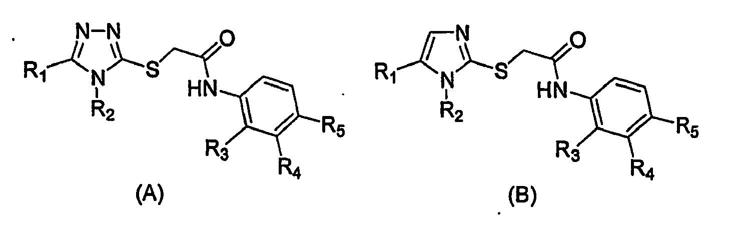 wherein R₁ is lower alkyl (optionally substituted), halogen or CF₃, R₂ is optionally substituted cycloalkyl, substituted or unsubstituted aryl, substituted or unsubstituted quinoline, or optionally substituted isoquinoline, and R₃, R₄, and R₅ are independently hydrogen, halogen, optionally substituted alkyl, S-alkyl, CF₃, heterocycle, NR'R", S(O)₂R', P(O)R'R", OP(O)R'R",or C(O)R', wherein R' and R" are independently NH₂, NHAlkyl, NHAcyl, NAlkylAcyl, N(Alkyl)₂, O-alkyl, acyl, aryl, alkyl, heterocycle, or R' and R" form a ring. R₃ and R₄ may be the same or different, or may even be linked together via a chain of two to four carbon atoms. Similarly, R₅ may be the same as R₃, but is more preferably different from R₃. For example, R₄ or R₅ may independently have a structure as shown below:

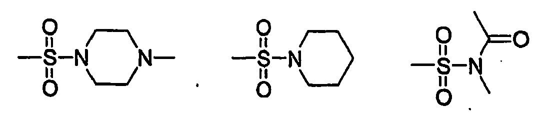

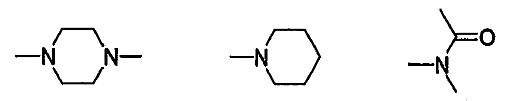

Thus, the ortho-substituted phenyl in the compounds according to Formulae A and B may further include at least one of a meta- and para-substituent (*e.g*., as defined as R₃ immediately above).

In especially preferred compounds according to Formulae A or B, R₂ is selected from the group consisting of a monosubstituted phenyl, a disubstituted phenyl, a trisubstituted phenyl, a monosubstituted naphthyl, a disubstituted naphthyl, a trisubstituted naphthyl, a monosubstituted quinoline, a disubstituted quinoline, a trisubstituted quinoline, a monosubstituted isoquinoline, a disubstituted isoquinoline, and a trisubstituted isoquinoline. Most preferably, the substituent(s) of the substituted aryl is an optionally substituted lower alkyl, CF₃, a lower alkoxy, a halogen, or NR'R", wherein R' and R" are independently H or lower alkyl.

Moreover, it should still further be recognized that in contemplated compounds the carboxamide group -C(Y)-NR₁R₂ may be replaced with an oxazole moiety. Such replacement may be advantageous to increase one or more pharmacokinetic/dynamic properties, and is thought to retaining the overall stereochemical configurations at least with respect to the atoms/groups interacting with the reverse transcriptase. Bioisosteric replacement approaches are described in George A. Patani and Edmond J. LaVoie, Bioisosterism: A rational approach in drug design, Chem. Rev. 1996, 96, 3147-3176, or in Preben H. Olsen, The use of bioisosteric groups in lead optimization, Current Opinion in Drug Discovery & Development 2001, 4, 471-478, both incorporated by reference herein.

Synthesis and modification of various oxazoles is described in Toshikazu Ibata and Yasushi Isogami, Formation and reaction of oxazoles. Synthesis of N-substituted 2-(aminomethyl)oxazoles, Bull. Chem. Soc. Jpn 1989, 62, 618-620, or in Toshikazu Ibata and Ryohei Sato, The acid catalyzed decomposition of diazo compounds. I. Synthesis of oxazoles in the BF3 catalyzed reaction of diazo carbonyl compounds with nitriles, Bull. Chem. Soc. Jpn 1979, 52, 3597-3600, and these and other references known in the art may be employed to provide some guidance for preparation of contemplated compounds in which the carboxamide group has been replaced with an oxazole moiety.

### Synthesis of Contemplated Compounds

It should be particularly appreciated that some of the contemplated compounds are commercially available from various sources, and all of the commercially available compounds are contemplated suitable for use herein. However, numerous of the contemplated compounds are not commercially available, and synthesis of some of those compounds may be performed following a protocol substantially as described in U.S. Pat. No. 5,939,462, which is incorporated by reference herein.

It should be recognized, however, that numerous alternative synthetic routes for the preparation contemplated compounds are also considered and the following exemplary routes are provided for guidance of a practitioner of ordinary skill in the art. For example, in one synthetic route, a suitably substituted amine (*e.g*., primary or secondary amine) is reacted with activated carbonyl containing compounds (preferably a carbonyl halide), wherein the carbonyl containing compound further includes a leaving group (and most preferably bromine). After formation of the carbonyl amide, the reaction product is reacted with a nucleophilic group (*e.g*., OH, SH, or NR₁R₂ with R₁ and R₂ independently hydrogen alkyl, etc. as outlined for Formula (I) above) of a second reagent thereby replacing the leaving group to form the desired compound as depicted in Scheme 1 below.

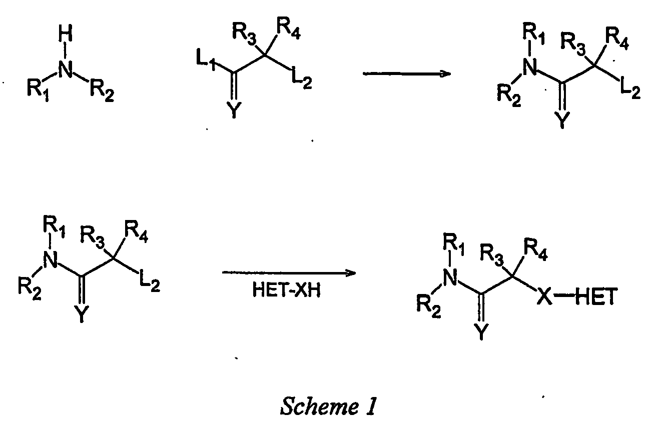

R₁ and R₂ of Scheme 1 may be any suitable substituent and is generally contemplated that appropriate R₁ and R₂ independently include hydrogen, substituted or unsubstituted alkyl, substituted or unsubstituted alkenyl, substituted or unsubstituted alkynyl, substituted or unsubstituted aryl, all of which may further include one or more heteroatoms. However, it is generally preferred that one of R₁ and R₂ is relatively small (*e.g*., hydrogen, methyl, trifluoromethyl, etc.), while the other of R₁ and R₂ comprises an aryl group. Especially preferred aryl groups will be substituted, most preferably in ortho-position, and may further include a substituent in para-position (*e.g*., ortho-substituted phenyl with halogen or methyl as substituent). Therefore, especially contemplated R₁ will include a hydrogen and lower alkyl (which may be further substituted), while R₂ may be selected from the group consisting of an aryl, a heteroaryl, a cycloalkyl, a cycloalkenyl, and a heterocycle.

Similarly, it is contemplated that the choice of leaving groups L₁ and L₂ will depend at least to some extent up on the particular choice of the amine and/or HET-XH, and all suitable leaving groups are contemplated. However, it is particularly preferred that L₁ and L₂ are a halide, and most preferably a bromide. Alternatively, L₁ may also be OH or O-Acyl. With respect to R₃ and R₄ the same considerations as described above for R₃ and R₄ in Formula (III). Y may be O, S, or NR with R as defined above. X in HET-XH is typically a heteroatom or CH₂, and most preferably S, S(O), S(O)₂, or O. HET may be any heterocycle, and particularly suitable heterocycles include those described above. Suitable solvents include ethers, alcohols, and hydrocarbons (optionally halogenated) and the choice of suitable solvents will at least in part depend on the chemical nature of the particular reagent. Furthermore with respect to the catalyst and/or base employed in the above reaction, the same considerations as those described by Connell et al. (U.S. Pat. No. 5,939,462) apply.

Alternatively, synthesis may follow a general protocol as outlined in **Scheme 2**, in which contemplated compounds are prepared from two separately prepared precursors. The first precursor comprising a substituted heterocycle may be prepared following a protocol similar to the protocols given below in the section entitled "Examples". Similarly, the second precursor comprising a substituted aryl may be prepared following a protocol similar to the protocols given below in the section entitled "Examples". Fusion of the so prepared precursors is typically carried out in DMF with potassium carbonate.

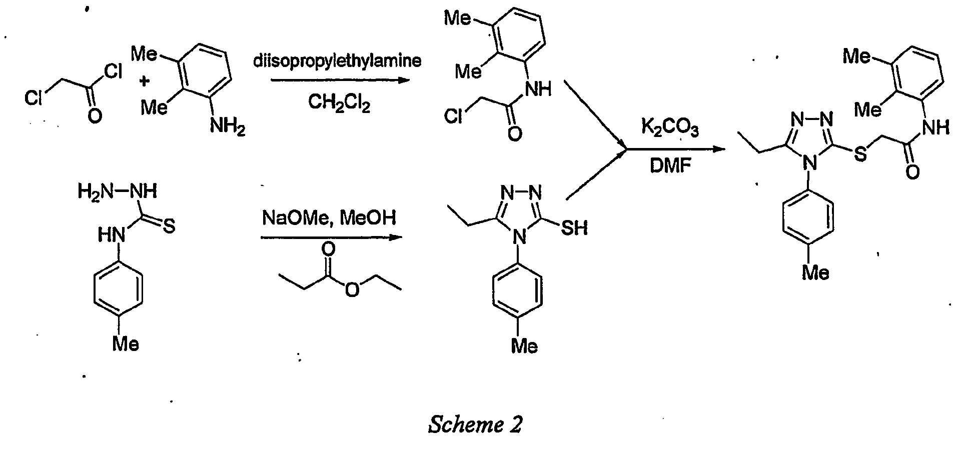

Where the substituted heterocycle is substituted with a heteroaryl or an aryl for which the corresponding thiosemicarbazide is not commercially available, and where the aryl comprises an ortho-substituted chlorophenyl, a synthetic procedure as described in **Scheme 3** below may be employed following procedures similar to the protocols given in the section entitled "Examples".

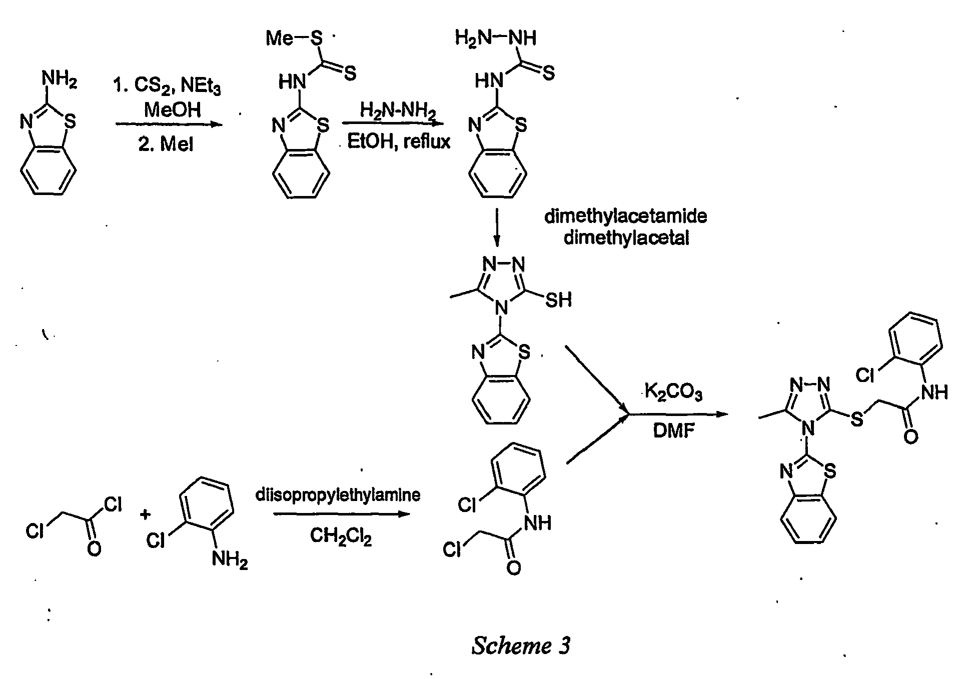

In yet another exemplary synthetic route, where the substituted heterocycle is substituted with an substituted aromatic group for which the corresponding amino form is not commercially available, a synthetic procedure as described in **Scheme 4** below may be employed, which follows a procedure similar to the protocols given below in the section entitled "Examples".

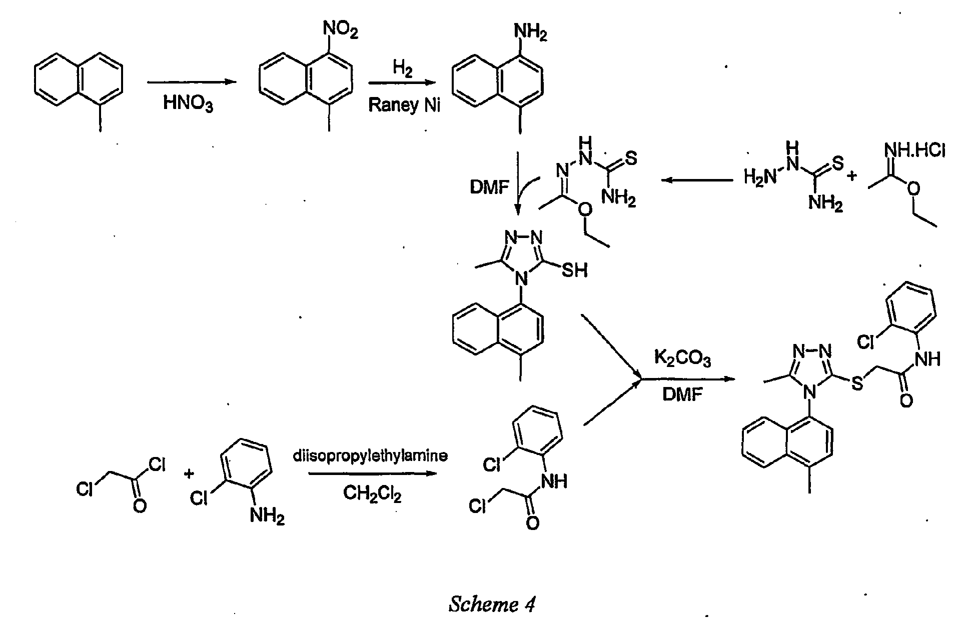

Alternatively, where the substituted heterocycle is substituted with a substituted heterocyclic aromatic group for which the corresponding amino is not commercially available, a synthetic procedure as described in **Scheme 5** below may be employed which substantially follows a procedure as in the protocols given below in the section entitled "Examples".

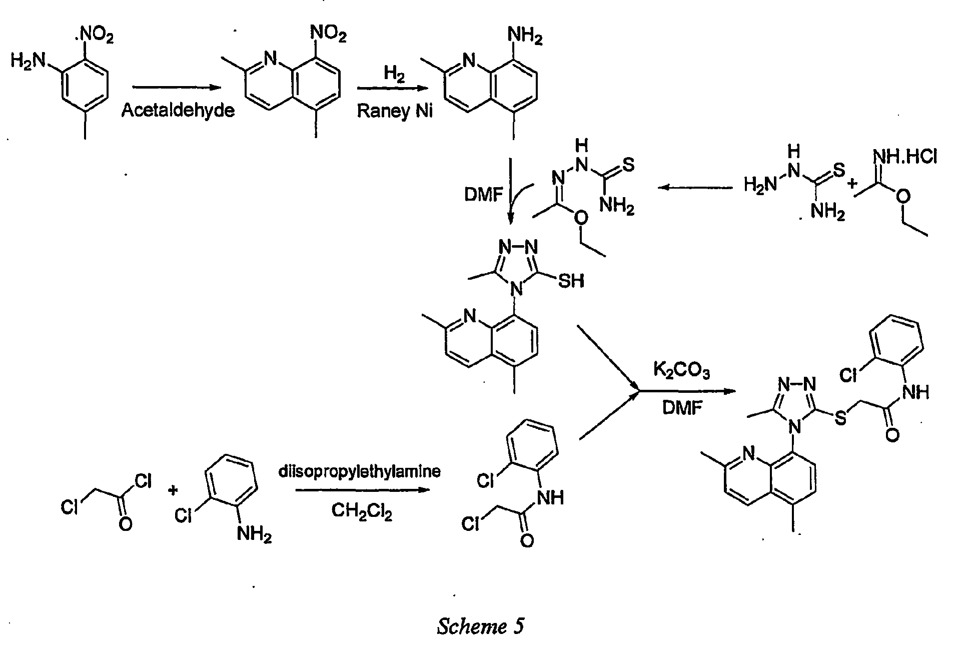

It should also be recognized that the carboxamide in the linker moiety may be prepared from a non-commercially available substituted aniline, and an exemplary synthetic procedure is described in **Scheme 6**, which substantially follows a procedure as in the protocols given below in the section entitled "Examples".

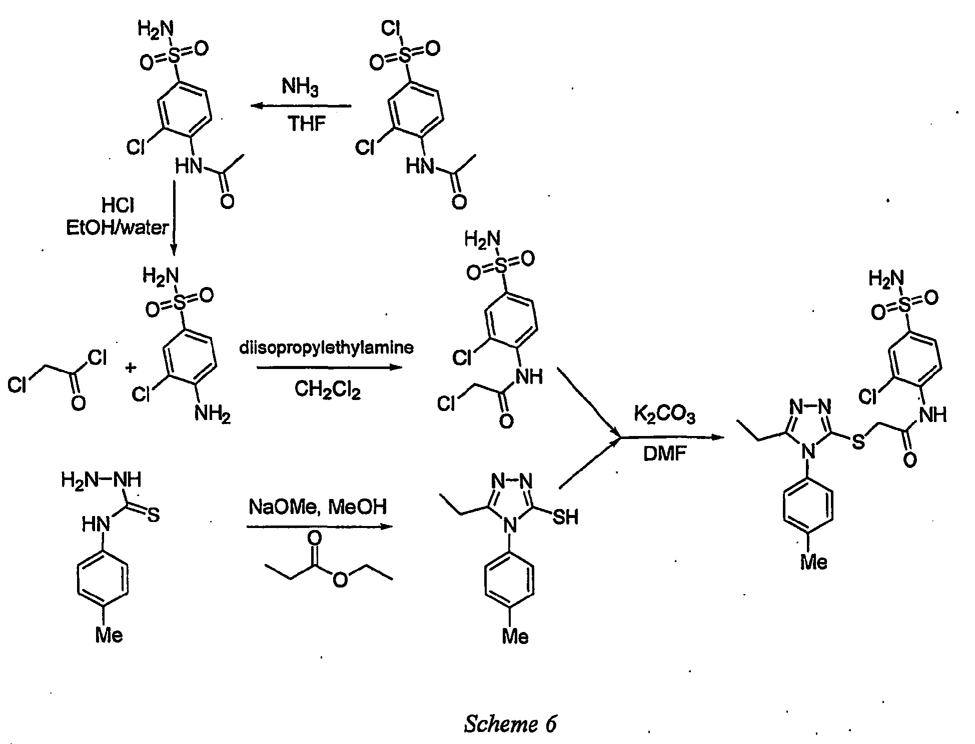

In still further contemplated exemplary routes to prepare the compounds according to the inventive subject matter, suitable HET groups may also be substituted with a halogen. One exemplary synthesis of compounds with such halogen-substituted HET moieties is described in **Scheme 7**, which substantially follows a procedure as in the protocols given below in the section entitled "Examples".

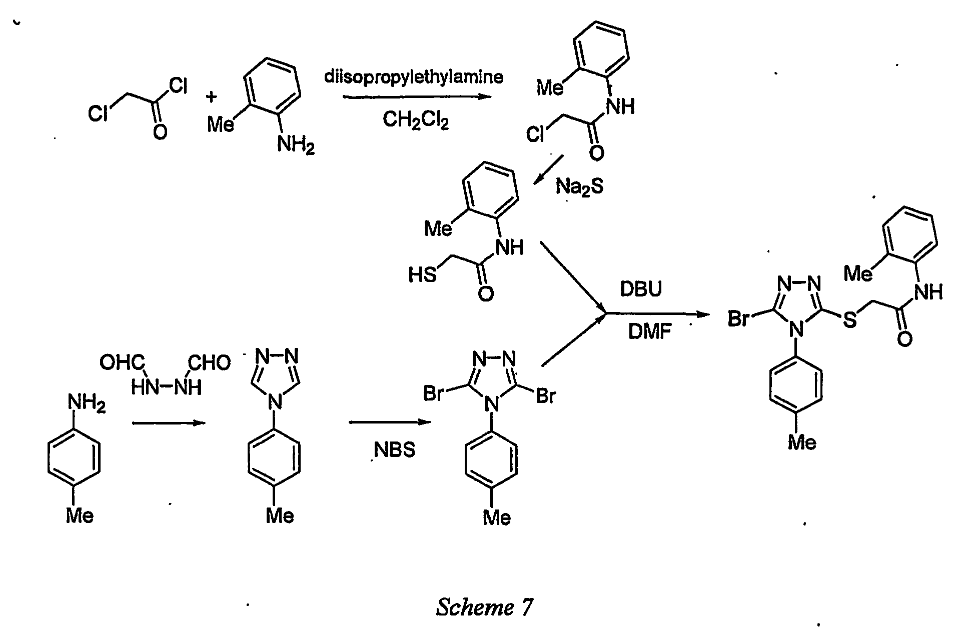

Where the heterocycle is a imidazole, a synthetic procedure as described in **Scheme 8** below may be employed which substantially follows a procedure as in the protocols given below in the section entitled "Examples".

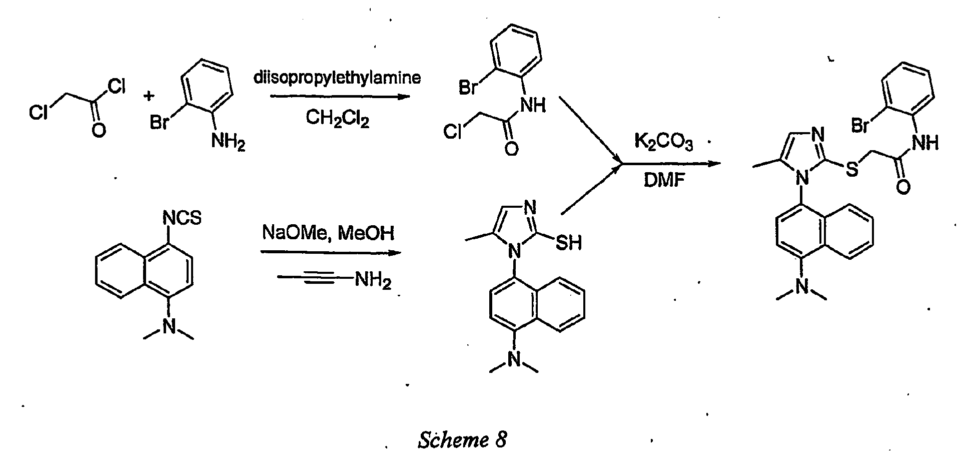

Alternatively, where the triazole is substituted with a CF₃, a synthetic procedure as described in **Scheme 9** below may be employed which substantially follows similar procedures as in the protocols given below in the section entitled "Examples".

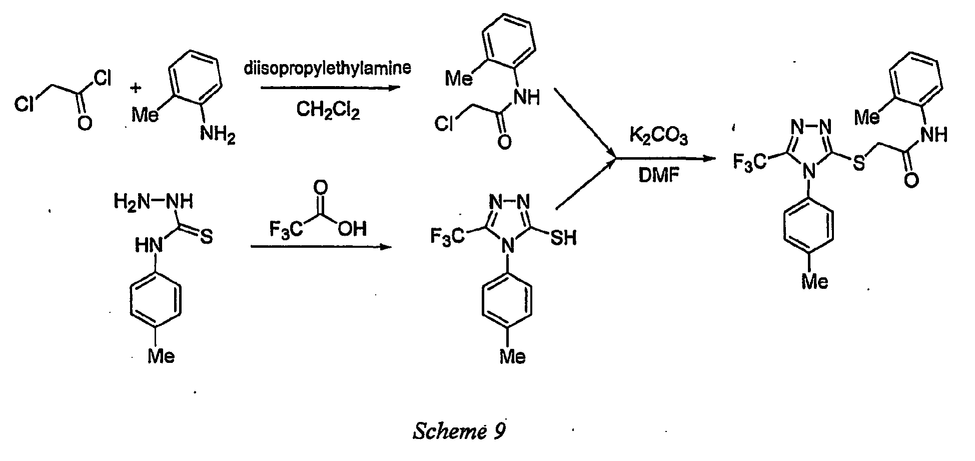

Where it is desirable that contemplated compounds include a oxazole group in place of a carboxamide group, synthesis may proceed as schematically depicted in **Scheme 10,** wherein the oxazole moiety may be formed on the group equivalent to the R₂ radical of Formula (I), and wherein the oxazole moiety with the R₂-equivalent radical is then covalently coupled to the substituted triazole heterocyclic base.

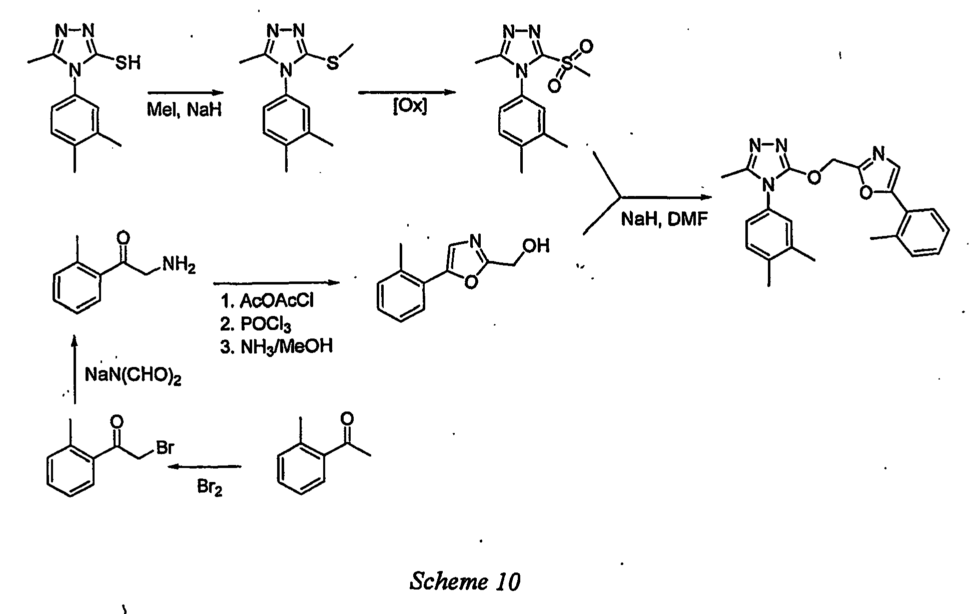

In still further contemplated aspects, and especially where contemplated compounds include an acid or a basic group, it should be appreciated that the corresponding salt (and preferably a pharmacologically acceptable salt) may be formed. For example, where contemplated compounds include a basic group, an acid addition salt may be prepared. Acid addition salts of such basic compounds can be prepared in a standard manner in a suitable solvent from the compound and an excess of acid, including hydrochloric, hydrobromic, sulfuric, phosphoric, acetic, maleic, succinic, or methanesulfonic acid. Likewise, if contemplated compounds include an acidic group, alkaline addition salts may be prepared (*e.g*., by treatment of the acidic compound with an excess of an alkaline reagent, such as hydroxide, carbonate or alkoxide, containing an appropriate cation. Suitable cations include Na⁺, K⁺, Ca²⁺, or NH₄⁺).

### Pharmaceutical Compositions comprising Contemplated Compounds

Where contemplated compounds are administered in a pharmacological composition, it is contemplated that suitable compounds can be formulated in admixture with a pharmaceutically acceptable carrier. For example, contemplated compounds can be administered orally as pharmacologically acceptable salts (see above), or intravenously in physiological saline solution (*e.g*., buffered to a pH of about 7.2 to 7.5). Conventional buffers such as phosphates, bicarbonates or citrates can be used for this purpose. Of course, one of ordinary skill in the art may modify the formulations within the teachings of the specification to provide numerous formulations for a particular route of administration. In particular, contemplated compounds may be modified to render them more soluble in water or other vehicle, which for example, may be easily accomplished by minor modifications (salt formulation, esterification, *etc*.) that are well within the ordinary skill in the art. It is also well within the ordinary skill of the art to modify the route of administration and dosage regimen of a particular compound in order to manage the pharmacokinetics of the present compounds for maximum beneficial effect in a patient.

In certain pharmaceutical dosage forms, prodrug forms of contemplated compounds may be formed for various purposes, including reduction of toxicity, increasing the organ-or target cell specificity, etc. One of ordinary skill in the art will recognize how to readily modify the present compounds to pro-drug forms to facilitate delivery of active compounds to a target site within the host organism or patient (see above). One of ordinary skill in the art will also take advantage of favorable pharmacokinetic parameters of the pro-drug forms, where applicable, in delivering the present compounds to a targeted site within the host organism or patient to maximize the intended effect of the compound.

In addition, contemplated compounds may be administered alone or in combination with other agents for the treatment of HIV, and particularly contemplated additional compounds include nucleoside-type reverse transcriptase inhibitors (*e.g*., Lamivudine, Zidovudine, Stavudine, Abacavir, Tenofovir or Didanosine), non-nucleoside reverse transcriptase inhibitors (*e.g*., Nevirapine, Delavirdine, Efavirenz), protease inhibitors (*e.g*., Sequinavir, Indinavir, Nelfinavir), a fusion inhibitor (*e.g*., Enfuvirtide), a CCR5 antagonist, immunotherapeutic agents (*e.g*., ribavirin, IL-2), an active, passive, and/or therapeutic vaccine. Combination therapies according to the present invention comprise the administration of at least one compound of the present invention or a functional derivative thereof and at least one other pharmaceutically active ingredient. The active ingredient(s) and pharmaceutically active agents may be administered separately or together and when administered separately this may occur simultaneously or separately in any order. The amounts of the active ingredient(s) and pharmaceutically active agent(s) and the relative timings of administration will be selected in order to achieve the desired combined therapeutic effect.

Therefore, the inventors contemplate that a pharmaceutical composition may comprise a compound of structure HET-L-C(Y)NR₁R₂, wherein HET comprises a preferably substituted heterocycle, L is a linker in which at least two atoms form a contiguous chain, wherein one of the two atoms is covalently bound to the heterocycle, and wherein another one of the two atoms is covalently bound to the carbonyl atom, Y is O, S, or NR₃, R₁ and R₃ are independently selected from the group consisting of hydrogen, halogen, and lower alkyl, R₂ is selected from the group consisting of a substituted or unsubstituted aryl, a cycloalkanyl, a cycloalkenyl, and a substituted or unsubstituted heterocycle, and wherein the compound is present in a concentration effective to inhibit a reverse transcriptase and/or HIV replication in a cell of a patient when administered to the patient.

With respect to suitable concentrations of contemplated compounds in pharmaceutical compositions, it should be appreciated that a person of ordinary skill in the art will readily adjust the amount of the compound to achieve inhibition of the reverse transcriptase and/or HIV replication. For example, inhibition of the HIV replication in a cell (typically a T-cell infected with the HIV virus) may be monitored *in vitro* using a blood culture and a luciferase based assay system as described below. Alternatively, inhibition of the reverse transcriptase may be monitored *in vivo* using RT-PCR to determine the amount of copies of viral DNA and/or RNA in blood or lymph nodes (containing HIV infected cells). However, it is generally contemplated that suitable concentrations will achieve a serum concentration of between 1 nM (in some cases even between 0.01 nM and 1 nM) and 100 microM.

In particularly preferred compounds, HET is a substituted triazole or imidazole, and it is even more preferred that the substituted triazole or imidazole is substituted with a first substituent (*e.g*., methyl, CF₃ or halogen) and a second substituent (*e.g*., toluyl, naphthyl, or quinoline), and wherein at least one of the first and second substituents includes a substituted phenyl group. Furthermore, it is generally preferred that the linker L has the structure -X₁-CR₃R₄-, wherein X₁ is selected from the group consisting of CH₂, S, O, S(O), S(O)₂, NH, NR₃ and CR₃R₄, and wherein R₃ and R₄ are independently hydrogen, halogen, lower alkyl, lower alkenyl, lower alkynyl, NH₂, OH, and SH. Thus, especially preferred linkers include those in which L is -S-CH₂-, -S(O)-CH₂-, -S(O)₂-CH₂-, -O-CH₂-, -NH-CH₂, -N(Me)-CH₂ or -CH₂-CH₂-. Moreover, particularly suitable substituents for the nitrogen atom R₁ and R₂ include hydrogen and a substituted aryl, respectively, and an especially preferred R₂ is an ortho-substituted phenyl (wherein the ortho-substituent is a halogen, a CF₃ or a methyl).

Consequently, particularly preferred pharmaceutical compositions will include contemplated compounds according to Structures A or B below: 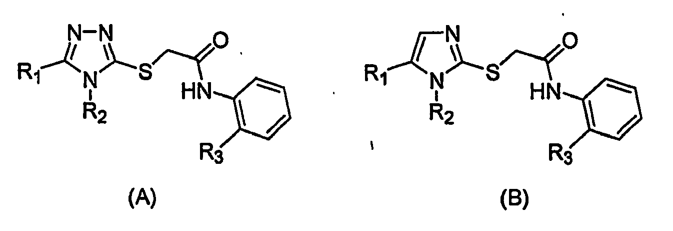 wherein R₁ is optionally substituted lower alkyl, CF₃, halogen, or hydrogen, wherein R₂ is cycloalkyl, substituted or unsubstituted aryl, substituted or unsubstituted heterocycle, and R₃ is lower alkyl or halogen.

In yet another aspect of the inventive subject matter, it should be recognized that contemplated compounds may be employed as a pharmaceutical product for treatment of a viral (and especially retroviral) infection in a mammal (typically human). Therefore, it is contemplated that suitable pharmaceutical products will include contemplated compounds, and an instruction to administer the compound to a patient infected with a retrovirus under a protocol that reduces viral propagation of the retrovirus. For example, the compounds may be provided in dosages for oral or parenteral administration (*infra*)*,* while suitable instructions to administer the compound will typically include a package insert or a prescription information. Alternatively, contemplated instructions may also include specific treatment schedules adapted to a particular treatment regimen (*e.g*., where contemplated compounds are co-administered in a combination therapy) or a sales brochure or other advertising publication. Protocols contemplated herein include all known forms of administrations to reduce the viral titer in the patient, or to even eliminate the virus from the patient entirely.

### Contemplated Use

The inventors surprisingly discovered (for experiments and data see below in the section with the title "Examples") that contemplated compounds exhibit significant *in vitro* and/or *in vivo* inhibitory effect on a reverse transcriptase, and especially on the reverse transcriptase of the HIV virus.

Consequently, the inventors contemplate a use for inhibiting a reverse transcriptase in which a reverse transcriptase is presented with a compound according to Formula (I)

HET-L-C(Y)NR₁R₂ (I)

wherein HET comprises a preferably substituted heterocycle; L is a linker in which at least two atoms form a contiguous chain, wherein one of the two atoms is covalently bound to the heterocycle, and wherein another one of the two atoms is covalently bound to the carbonyl carbon atom; Y is O, S, or NR₃; R₁ and R₃ are independently selected from the group consisting of hydrogen, halogen, and optionally substituted lower alkyl; and R₂ is selected from the group consisting of a substituted or unsubstituted aryl, a cycloalkyl, a cycloalkenyl, and a substituted or unsubstituted heterocycle.

In particularly preferred aspects of contemplated uses, the heterocycle comprises a nitrogen-containing heterocycle, and is most preferably substituted triazole or imidazole. While the substituent or substituents on contemplated heterocycles may vary considerably, it is generally preferred that the substituted triazole or imidazole will include a first and second substituent, wherein the first substituent is relatively small (*e.g*., methyl, trifluoromethyl, nitro, amino, halogen, hydroxy, or thio group) and wherein the second substituent includes an aromatic system (and most preferably a substituted naphthyl, substituted phenyl or substituted quinoline). With respect to the aromatic system, the inventors discovered that where the aromatic system comprises a phenyl group, particularly strong inhibition could be achieved where the phenyl group has a substituent in the ortho-position. The same observations were made for compounds having a naphthyl group or a quinoline group.

Furthermore, it is contemplated that the nature and particular structure of the linker connecting the heterocycle to the carbonyl carbon may vary considerably, and it is generally contemplated that the linker may allow steric flexibility or may orient the heterocycle in a relatively fixed position relative to the carbonyl group. For example, where the linker is relatively flexible, it is contemplated that all of covalent bonds between the atoms that form a contiguous chain to connect the heterocycle with the carbonyl carbon are single bonds. Of course, it should be recognized that the bond angle between such atoms will depend at least to some degree on the chemical nature of the atoms. Therefore, relatively straight angles (*e.g*., where the atom is O or S) are contemplated as well as non-straight angles (*e.g*., where the atom is C or P).

On the other hand, where the linker is relatively rigid, suitable linkers may include two or more atoms (within the contiguous chain of atoms that connect the heterocycle with the carbonyl carbon) that are covalently coupled to each other via a double or triple bond. Such linkers may therefore include unsaturated straight or branched hydrocarbons chains, or aromatic rings. Alternatively, contemplated linkers may also include cycloalkyl groups. Moreover, suitable linkers may further include various functional groups to provide particular physicochemical properties, including a hydrogen bond donor or acceptor group, a polar or non-polar group, an ionic group, or a lipophilic group. Thus, suitable linkers may include between 2 and 20 (and even more) atoms; which may or may not include heteroatoms.

Consequently, particularly preferred linkers may have the structure -X₁-CR₃R₄-, wherein X₁ is selected from the group consisting of S, O, S(O), S(O)₂, NH, NR₃ and CR₃R₄, and wherein R₃ and R₄ are independently hydrogen, halogen, lower alkyl, lower alkenyl, lower alkynyl, NH₂, OH, and SH. Even more preferred linkers will include those selected from the group of -S-CH₂-, -S(O)-CH₂-, -S(O)₂-CH₂-, -O-CH₂-, -NH-CH₂-, -N(Me)CH₂-and -CH₂-CH₂-.

In yet further aspects of preferred uses, the carbonyl carbon may be covalently bound to an oxygen, sulfur, or a NH or NR group, wherein R may be selected from the group consisting of hydrogen, halogen, and lower alkyl. Consequently, contemplated compounds may include a carboxamide group, a (substituted) carboxamidine group, or a thiocarboxamide group.

In still further aspects of preferred uses, R₁ and R₂ may vary considerably, and all R₁ and R₂ groups contemplated above in the section entitled "Contemplated Compounds" are considered suitable for use herein. However, it is generally preferred that R₁ is hydrogen and R₂ is a substituted aryl (and most preferably that R₂ comprises an ortho-substituted phenyl, wherein the ortho-substituent is a halogen, a SCH₃, a CF₃ or a methyl).

It should further be appreciated that contemplated uses for inhibition of a reverse transcriptase need not be limited to a particular reverse transcriptase, and it should be recognized that all known reverse transcriptases are considered suitable for use herein. However, it is particularly preferred that the reverse transcriptase is a viral reverse transcriptase, and in especially preferred aspects the viral reverse transcriptase is from HIV. Moreover, the inventors discovered that such reverse transcriptases may be inhibited even when the reverse transcriptase is at least partially resistant to a non-nucleoside analog reverse transcriptase inhibitor. The term "at least partially resistant to a non-nucleoside analog reverse transcriptase inhibitor" as used herein means that the 'at least partially resistant' reverse transcriptase is inhibited by previously known non-nucleoside reverse transcriptase inhibitors to a lesser degree than a non-resistant reverse transcriptase (see section with the title "Examples").

With respect to the step of presenting the reverse transcriptase, it is contemplated that all manners of presentation are suitable and include numerous *in vitro* and *in vivo* presentations. For example, where presentation of the reverse transcriptase with contemplated compounds is *in vitro,* it should be appreciated that the reverse transcriptase may be in a solvent or supported on a solid phase (and optionally in the presence of an RNA or DNA template, cofactors, and nucleotides, etc.). Contemplated solvents include those that are predefined (*e.g*., reverse transcriptase buffer) as well as those where the exact chemical composition is highly complex (*e.g*., cell lysate). Suitable solid phases include gels, polymer beads, walls of a microplate, etc. Furthermore, particularly contemplated *in vitro* presentation also includes a presentation where the reverse transcriptase is enclosed by a cell (infected by the HIV virus, or transfected and transformed to produce recombinant reverse transcriptase), and wherein the cell is in an environment that includes contemplated compounds.

Thus, *in vitro* presentation includes all manners of presentation in which the reverse transcriptase is in the same environment as contemplated compounds. Consequently, contemplated compounds may be added to a buffer, medium, or other solvent in which the reverse transcriptase is present, and addition of contemplated compounds includes addition in dissolved form as well as in solid form. With respect to the particular form (*e.g*., as solution in a particular solvent) in which contemplated compounds are added to the environment, a person of ordinary skill in the art will readily determine a suitable form. Similarly, the appropriate concentration may readily be determined by a person of ordinary skill in the art without undue experimentation (*e.g*., using IC₅₀ data as guidance).

Similarly, contemplated *in vivo* presentations include all manners of adding contemplated compounds in a suitable formulation to an environment that contains the reverse transcriptase, and especially contemplated environments include mammals infected with a retrovirus, and most preferably the HIV virus. Consequently, particularly preferred *in vivo* presentations include administration of pharmaceutical compositions comprising contemplated compounds to a patient that is infected with the HIV virus. Thus, suitable administration may be oral and/or parenteral (systemic) administration as well as *ex vivo* administration to whole blood or components thereof with reintroduction of at least a portion of the whole blood or components thereof. Exemplary pharmaceutical compositions are described above in the section with the title "Pharmaceutical Compositions comprising Contemplated Compound".

Therefore, the inventors contemplate a use for treating an HIV infected patient in which a pharmaceutical composition comprising a compound according to Formula (I) is administered to the patient at a dosage effective to reduce viral propagation, wherein Formula (I) is HET-L-C(Y)NR₁R₂, in which HET comprises a preferably substituted heterocycle, L is a linker in which at least two atoms form a contiguous chain, wherein one of the two atoms is covalently bound to the heterocycle, and wherein another one of the two atoms is covalently bound to the carbonyl atom, Y is oxygen, sulfur, NH, or NR (with R as described above), R₁ is selected from the group consisting of hydrogen, halogen, and methyl, and R₂ is selected from the group consisting of a substituted or unsubstituted aryl, a cycloalkanyl, a cycloalkenyl, and a substituted or unsubstituted heterocycle. With respect to particularly preferred structures, the same considerations as described above in the section entitled "Contemplated Compounds" apply.

Therefore, particularly preferred compounds for treatment of an HIV infected patient include those in which HET is a substituted triazole or imidazole, and/or L is selected from the group consisting of -S-CH₂-, -S(O)-CH₂-, -S(O)₂-CH₂-, -O-CH₂-, -NH-CH₂-, - N(Me)CH₂- and -CH₂-CH₂-, and in which Y is oxygen. In still further preferred compounds for treatment methods, R₁ is hydrogen and R₂ is a substituted aryl. Thus, particularly preferred compounds for treatment of an HIV infection include compounds of structures A or B 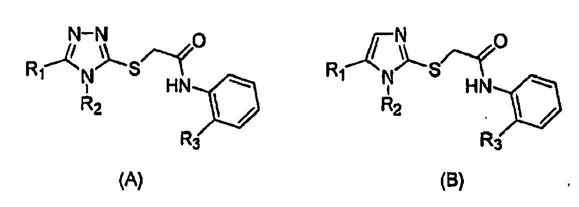 wherein R₁ is lower alkyl., halogen or CF₃, R₂ is cycloalkyl, substituted aryl, or unsubstituted aryl, substituted quinoline or unsubstituted quinoline and R₃ is lower alkyl, S-alkyl, CF₃ or halogen. With respect to the dosage, it is contemplated that the dosage will predominantly depend on the particular compound employed (*e.g*., particular solubility, efficacy, bioavailability and/or metabolic profile), and it should be recognized that a person of ordinary skill in the art will readily be able to determine the proper dosage or dosage range. Similarly, reduction of viral propagation may be monitored using various methods well known in the art. For example, viral propagation may be measured using quantitative RT-PCR to determine the number of viral copies in a particular biological sample (e.g., whole blood).

Of course, it should be recognized that, where desirable, contemplated compounds may be converted into a prodrug form to increase specificity towards an infected cell, to reduce adverse activity in non-infected cells, to increase bioavailability, etc., and suitable administration formulations, routes, and protocols are well known in the art (see also above). Exemplary suitable protocols for conversion of contemplated compounds into the corresponding prodrug form can be found in Francisca Lopez, Rui Moreira and Jim Iley, Acyloxymethyl as a drug protecting group. Part 6: N-acyloxymethyl- and N-[(aminocarbonyloxy)methyl]sulfonamides as prodrugs of agents containing a secondary sulfonamide group, Bioorg. Med. Chem. 2000, 8, 707-716, or Jom Drustrup Larsen and Hans Bundgaard, Prodrug forms for the sulfonamide group. I. Evaluation of N-acyl derivatives, N-sulfonylamidines, N-sulfonylfilimines and sulfonylureas as possible prodrug derivatives, International Journal of Pharmaceutics, 1987, 37, 87-95, or in Joseph H. Chan, Benzophenones as inhibitors of reverse transcriptase, WO 02/070470, all of which are incorporated by reference herein.

### Examples

The following experiments are provided only to illustrate exemplary aspects of the inventive subject matter and should not be understood as limiting the inventive subject matter.

### N-(2-Bromo-4-methylphenyl)-2-(5-methyl-4-phenyl-4H-[1,2,4]triazole-3-ylsulfanyl)-acetamide

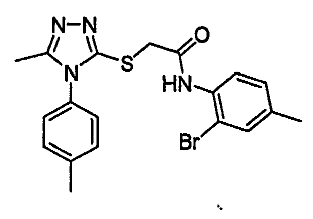

5-Methyl-4-phenyl-4H-1,2,4-triazole-3-thiol: A suspension of 4-phenyl-3-thiosemicarbazide (10 g, 59.8 mmol) in dimethyl acetamide dimethyl acetal (30 mL, 205 mmol) was heated in an open flask on a steam bath for 1.5 h. Removal of the solvent and flash chromatography of the residue (2% methanol/dichloromethane) affords a mixture of 5-methyl-4-phenyl-4H-1,2,4-triazole-3-thiol and 3-methyl-5-methylthio-4-phenyl-4H-1,2,4-triazole.

N-(2-Bromo-4-methylphenyl)-2-chloroacetamide: 2-Bromo-4-methylphenyl (500 mg, 2.69 mmol) was added to a mixture of chloroacetylchloride (0.14 mL, 2.69 mmol) and diisopropylmethylamine (0.47 mL, 2.69 mmol) in dichloromethane (16 mL). After 4 hours of stirring the mixture was diluted with ethyl acetate and washed with 1 N hydrochloric acid, water, saturated aqueous sodium chloride solution, and dried over MgSO₄. Removal of the solvent in vacuo affords the desired compound.

N-(2-Bromo-4-methylphenyl)-2-(5-methyl-4-phenyl-4H-[1,2,4]triazole-3-ylsulfanyl)acetamide: A mixture of 5-Methyl-4-phenyl-4H-1,2,4-triazole-3-thiol (200 mg, 1.05 mmol), potassium carbonate (153.6 mg, 1.1 mmol), and N-(2-Bromo-4-methylphenyl)-2-chloroacetamide (273.5 mg, 1.05 mmol) in N,N-dimethylformamide (5 mL) was stirred overnight. The resulting mixture was diluted with water and extracted with ethyl acetate. The organic layer was washed with water, saturated aqueous sodium chloride solution, and dried over MgSO₄. Removal of the solvent *in vacuo* and flash chromatography of the residue affords the desired compound.

### N-(2-Bromo-phenyl)-2-[5-methyl-4-(4-methyl-naphthalen-1-yl)-4H-[1,2,4]triazol-3-ylsulfanyl]-acetamide

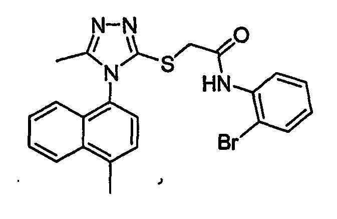

4-Methyl-nitronaphthalene: Nitric acid (9.6 mL) was added slowly to neat 1-methylnaphthalene (3 g, 21 mmol) at 0°C. Water (20 mL) was then added and the aqueous layer was extracted with benzene (40 mL). The organic layer was washed sodium hydroxide, dried over sodium sulfate and concentrated in vacuo. The resulting residue was purified by column chromatography (90% hexane /10% ethylacetate) to afford 2.82g of product as a yellow solid (72% yield).

4-Methyl-aminonaphthalene: To a solution of 4-methyl-nitronaphthalene (1 g, 5.3 mmol) in ethanol (80 mL), was added Raney Ni (0.9 g) and the mixture stirred 4 h. under hydrogen (5 psi). The catalyst was then filtered out and the solvent remove in vacuo to give the crude title compound (772 mg, 92% yield) as a yellow oil which was used in the next step without further purification.

Ethyl Acetimidate Thiosemicarbazone: To a solution of ethyl acetimidate hydrochloride (1 g, 8.1 mmol) in dimethyl formamide (16 mL) was added thiosemicarbazide (738 mg, 8.1 mmol) and the mixture stirred at room temperature for 1 h. Water was then added to the reaction until a precipitate (product) was formed (1.16 g, 89%).

5-Methyl-4-(4-methyl-naphthalen-1-yl)-4H-[1,2,4]triazole-3-thiol: A solution of ethyl acetimidate thiosemicarbazone (488 mg, 3.02 mmol), dimethyl formamide (4 mL) and 4-methyl-aminonaphthalene (475 mg, 3.02 mmol) was heated at reflux for 3 hours. After evaporation of the solvent, a solution of 1 *N* sodium hydroxide was added (10 mL) and the mixture was stirred for 20 min at 40 °C. The reaction mixture was then extracted with ether to remove side products. The resulting aqueous layer was treated with a 10% solution of hydrochloric acid until the product precipitated (435 mg, 56%).

N-(2-Bromo-phenyl)-2-[5-methyl-4-(4-methyl-nap'hthalen-1-yl)-4H-[1,2,4]triazol-3-ylsulfanyl]-acetamide: To a solution of 5-methyl-4-(4-methyl-naphthalen-1-yl)-4H-[1,2,4]triazole-3-thiol (50 mg, 0.19 mmol) in dimelthyl formamide (1.5 mL) was added N-(2-Bromo-phenyl)-2-chloro-acetamide (prepared from 2-bromo aniline) (49 mg, 0.19 mmol) and potassium carbonate (30 mg, 0.22 mmol) and the mixture stirred 18 h at room temperature. Water was added and the aqueous layer extracted with ethyl acetate. The organic layer was dried over sodium sulfate and concentrated. The resulting residue was purified by column chromatography (90% dichloromethane/10% methanol) to afford 65 mg of title compound, 87% yield

### N-(2-Chloro-pyridin-3-yl)-2-[4-(4-ethyl-naphthalen-l-yl)-5-methyl-4H-[1,2,4]triazol-3-ylsulfanyl]-acetamide

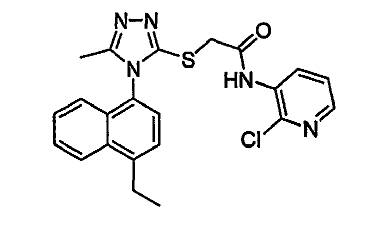

4-Ethyl-nitronaphthalene: Nitric acid (9.6 mL) was added slowly to neat 1-ethylnaphthalene (3 g, 19.2 mmol) at 0°C. Water (20 mL) was then added and the aqueous layer was extracted with benzene (40 mL). The organic layer was washed sodium hydroxide, dried over sodium sulfate and concentrated in vacuo. The resulting residue was purified by column chromatography (90% hexane /10% ethylacetate) to afford 3.2 of the title product as a yellow solid (84% yield).

4-Ethyl-aminonaphthalene: To a solution of 4-ethyl-nitronaphthalene (1 g) 4.9 mmol) in ethanol (80 mL), was added Raney Ni (0.9 g) and the mixture stirred 4 h. under hydrogen (5 psi). The catalyst was then filtered out and the solvent remove in vacuo to give a yellow oil which was purified by column chromatography (95% hexane/5% ethyl acetate) to afford the title product as a yellow solid (800 mg, 94% yield).

5-Methyl-4-(4-ethyl-naphthalen-1-yl)-4H-[1,2,4]triazole-3-thiol: A solution of 3 (1.94 g, 12.0 mmol), dimethyl formamide (10 mL)) and 7 (1.35 g, 12.0 mmol) was heated at reflux for 3 hours. After evaporation of the solvent, a solution of 1 *N* sodium hydroxide was added (25 mL) and the mixture was stirred for 20 min at 40 °C. The reaction mixture was then extracted with ether to remove side products. The resulting aqueous layer was treated with a 10% solution of hydrochloric acid until the desired product precipitated (1.48 g, 46%).

N-(2-Chloro-pyridin-3-yl)-2-[4-(4-ethyl-naphthalen-1-yl)-5-methyl-4H-[1,2,4]triazol-3-ylsulfanyl]-acetamide: To a solution of 5-methyl-4-(4-ethyl-naphthalen-1-yl)-4H-[1,2,4]triazole-3-thiol (50 mg, 0.19 mmol) in dimelthyl formamide (1.5 mL) was added N-(2-chloro-pyridin-3-yl)-2-chloro-acetamide (prepared from 2-Chloro-pyridin-3-ylamine) (39 mg, 0.19 mmol) and potassium carbonate (28 mg, 0.22 mmol) and the mixture stirred 18 h at room temperature. Water was added and the aqueous layer extracted with ethyl acetate. The organic layer was dried over sodium sulfate and concentrated. The resulting residue was purified by column chromatography (90% dichloromethane/10% methanol) to afford the desired product (86 mg, 93% yield).

### N-(2-Chloro-pyridin-3-yl)-2-[5-methyl-4-(4-methyl-5,6,7,8-tetrahydro-naphthalen-1-yl)-4H-[1,2,4]triazol-3-ylsulfanyl]-acetamide

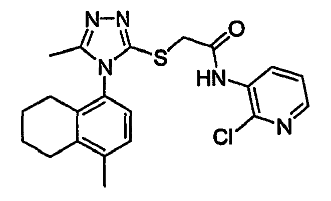

4-Methyl-5,6,7,8-tetrahydro-naphthalen-1-ylamine: To a solution of 4-methyl-nitronaphthalene (1.1 g, 5.9 mmol) in ethanol (80 mL), was added Raney Ni (0.9 g) and the mixture stirred 3 days under hydrogen (50 psi). The catalyst was then filtered out and the solvent remove in vacuo to give the crude desired compound (750 mg, 79% yield) as a yellow oil which was used in the next step without further purification.

5-Methyl-4-(4-methyl-5,6,7,8-tetrahydro-naphthalen-1-yl)-4H-[1,2,4]triazole-3-thiol: A solution of ethyl acetimidate thiosemicarbazone (500 mg, 3.1 mmol), dimethyl formamide (4 mL) and 4-methyl-aminonaphthalene (500 mg, 3.1 mmol) was heated at reflux for 3 hours. After evaporation of the solvent, a solution of 1 *N* sodium hydroxide was added (10 mL) and the mixture was stirred for 20 min at 40 °C. The reaction mixture was then extracted with ether to remove side products. The resulting aqueous layer was treated with a 10% solution of hydrochloric acid until the product precipitated (349.4 mg, 43.5%).

N-(2-Chloro-pyridin-3-yl)-2-[5-methyl-4-(4-methyl-5,6,7,8-tetrahydro-naphthalen-1-yl)-4H-[1,2,4]triazol-3-ylsulfanyl]-acetamide: To a solution of 5-methyl-4-(4-methyl-5,6,7,8-tetrahydro-naphthalen-1-yl)-4H-[1,2,4]triazole-3-thiol (50 mg, 0.19 mmol) in dimethyl formamide (1.5 mL) was added N-(2-chlbro-pyridin-3-yl)-2-chloro-acetamide (prepared from 2-Chloro-pyridin-3-ylamine) (39 mg, 0.19 mmol) and potassium carbonate (28 mg, 0.22 mmol) and the mixture stirred 18 h at room temperature. Water was added and the aqueous layer extracted with ethyl acetate. The organic layer was dried over sodium sulfate and concentrated. The resulting residue was purified by column chromatography (90% dichloromethane/10% methanol) to afford the desired compound (35 mg, 40% yield).

### N-(2-Bromo-phenyl)-2-[4-(2,4-dimethyl-naphthalen-1-yl)-5-methyl-4H-[1,2,4]triazol-3-ylsulfanyl]-acetamide

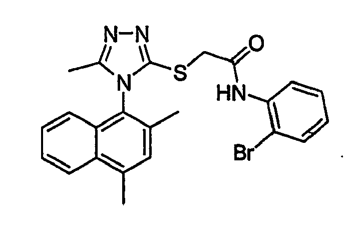

2,4-Dimethyl-1-nitro-naphthalene: Nitric acid (9.6 mL) was added slowly to neat 2,4-dimethyl-naphthalene (3 g, 19.2 mmol) at 0 °C. Water (20 mL) was then added and the aqueous layer was extracted with benzene (40 mL). The organic layer was washed sodium hydroxide, dried over sodium sulfate and concentrated in vacuo. The resulting residue was purified by column chromatography (90% hexane /10% ethylacetate) to afford 3.459 g of 13 as a yellow solid (89% yield).

2,4-Dimethyl-naphthalen-1-ylamine: To a solution of 2,4-dimethyl-1-nitronaphthalene (1 g, 4.9 mmol) in ethanol (80 mL), was added Raney Ni (0.9 g) and the mixture stirred 18 h under hydrogen (atmospheric pressure). The catalyst was then filtered out and the solvent remove in vacuo to give the crude desired compound which was purified by column chromatography (90% hexane/10% ethyl acetate) to give the pure desired amine (822 mg 97% yield).

4-(2,4-Dimethyl-naphthalen-1-yl)-5-methyl-4H-[1,2,4]triazole-3-thiol: A solution of ethyl acetimidate thiosemicarbazone (500 mg, 3.1 mmol), dimethyl formamide (4 mL) and 2,4-dimethyl-naphthalen-1-ylamine (534 mg, 3.1 mmol) was heated at reflux for 3 hours. After evaporation of the solvent, a solution of 1 *N* sodium hydroxide was added (10 mL) and the mixture was stirred for 20 min at 40 °C. The reaction mixture was then extracted with ether to remove side products. The resulting aqueous layer was treated with a 10% solution of hydrochloric acid until the product precipitated (284 mg, 34%).

N-(2-Bromo-phenyl)-2-[4-(2,4-dimethyl-naphthalen-1-yl)-5-methyl-4H-[1,2,4]triazol-3-ylsulfanyl]-acetamide: To a solution of 4-(2,4-dimethyl-naphthalen-1-yl)-5-niethyl-4H-[1,2,4]triazole-3-thiol (50 mg, 0.19 mmol) in dimethyl formamide (1.5 mL) was added N-(2-Bromo-phenyl)-2-chloro-acetamide (prepared from 2-bromo aniline) (47 mg, 0.19 mmol) and potassium carbonate (28 mg, 0.21 mmol) and the mixture stirred 18 h at room temperature. Water was added and the aqueous layer extracted with ethyl acetate. The organic layer was dried over sodium sulfate and concentrated. The resulting residue was purified by Column chromatography (90% dichloromethane/10% methanol) to afford the desired compound (49 mg, 58% yield).

### N-(2-Chloro-4-sulfamoyl-phenyl)-2-[4-(4,7-dimethyl-naphthalen-1-yl)-5-methyl-4H-[1,2,4]triazol-3-ylsulfanyl]-acetamide

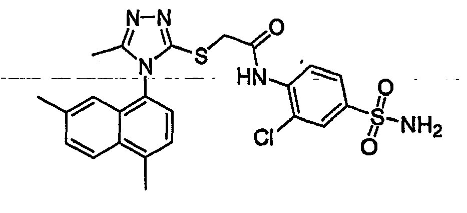

4,7-Dimethyl-1-nitro-naphthalene: Nitric acid (5 mL) was added slowly to neat 1,6-dimethyl-naphthalene (1 g, 6.4 mmol) at 0 °C. Water (20 mL) was then added and the aqueous layer was extracted with benzene (40 mL). The organic layer was washed sodium hydroxide, dried over sodium sulfate and concentrated in vacuo. The resulting residue was purified by column chromatography (90% hexane /10% ethylacetate) to afford 1.0 g of the desired product as a yellow solid (78% yield).

4,7-Dimethyl-naphthalen-1-ylamine: To a solution of 4,7-dimethyl-1-nitro-naphthalene (1 g, 4.9 mmol) in ethanol (80 mL), was added Raney Ni (0.9 g) and the mixture stirred 18 h. under hydrogen (atmospheric pressure). The catalyst was then filtered out and the solvent remove in vacuo. The crude mixture was purified by column chromatography (90% hexane/10% ethyl acetate) to give (434 mg 52% yield) of the pure desired amine.

4-(4,7-Dimethyl-naphthalen-1-yl)-5-methyl-4H-[1,2,4]triazole-3-thiol: A solution of ethyl acetimidate thiosemicarbazone (400 mg, 2.48 mmol), dimethyl formamide (6 mL) and 4,7-dimethyl-naphthalen-1-ylamine (425 mg, 2.48 mmol) was heated at reflux for 3 hours. After evaporation of the solvent, a solution of 1 *N* sodium hydroxide was added (10 mL) and the mixture was stirred for 20 min at 40 °C. The reaction mixture was then extracted with ether to remove side products. The resulting aqueous layer was treated with a 10% solution of hydrochloric acid until the desired product precipitated (370 mg, 55% yield)

N-(2-Chloro-4-sulfamoyl-phenyl)-2-[4-(4,7-dimethyl-naphthalen-1-yl)-5-methyl-4H-[1,2,4]triazol-3-ylsulfanyl]-acetamide: To a solution of 4-(4,7-dimethyl-naphthalen-1-yl)-5-methyl-4H-[1,2,4]triazole-3-thiol (50 mg, 0.19 mmol) in dimethyl formamide (1.5 mL) was added N-(2-Chloro-4-sulfamoyl-phenyl)-2-chloro-acetamide (44 mg, 0.19 mmol) and potassium carbonate (28 mg, 0.20 mmol) and the mixture stirred 18 h at room temperature. Water was added and the aqueous layer extracted with ethyl acetate. The organic layer was dried over sodium sulfate and concentrated. The resulting residue was purified by column chromatography (90% dichloromethane/10% methanol) to afford the desired product (34.3 mg, 39% yield).

### N-(2-Chloro-pyridin-3-yl)-2-[4-(2,5-dimethyl-quinolin-8-yl)-5-methyl-4H-[1,2,4]triazol-3-ylsulfanyl]-acetamide

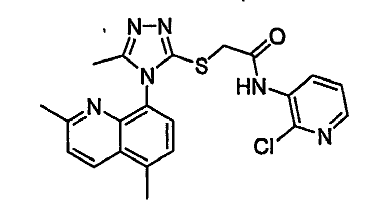

2,5-Dimethyl-8-nitro-quinoline: To a suspension of 2-nitro-5-methyl aniline (3 g, 19.8 mmol) in hydrochloric acid (12 mL) was added at room temperature acetaldehyde drop-wise and the mixture stirred at this temperature for 15 min and at 68°C for an additional hour. The reaction mixture was the cooled to room temperature, poured into iced water and neutralized with ammonium hydroxide to form a yellow precipitate (3.64 g, 89% yield).

2,5-Dimethyl-quinolin-8-ylamine: To a solution of 2,5-dimethyl-8-nitro-quinoline (2 g, 9.9 mmol) in ethanol (160 mL), was added Raney Ni (1.8 g) and the mixture stirred 18 h under hydrogen (atmospheric pressure). The catalyst was then filtered out and the solvent remove in vacuo. The crude mixture was purified by column chromatography (90% hexane/10% ethyl acetate) to give (1.5 g 88% yield) of the pure desired amine.

4-(2,5-Dimethyl-quinolin-8-yl)-5-methyl-4H-[1,2,4]triazole-3-thiol: A solution ethyl acetimidate thiosemicarbazone (1.7 g, 10.7 mmol), dimethyl formamide (12 mL) and 2,5-dimethyl-quinolin-8-ylamine (1.8 g, 10.7 mmol) was heated at reflux for 3 hours. After evaporation of the solvent, a solution of 1 *N* sodium hydroxide was added (10 mL) and the mixture was stirred for 20 min at 40 °C. The reaction mixture was then extracted with ether to remove side products. The resulting aqueous layer was treated with a 10% solution of hydrochloric acid until the product precipitated (465 mg, 17% yield).

N-(2-Chloro-pyridin-3-yl)-2-[4-(2,5-dimethyl-quinolin-8-yl)-5-methyl-4H-[1,2,4]triazol-3-ylsulfanyl]-acetamide: To a solution of 4-(2,5-dimethyl(quinolin-8-yl)-5-methyl-4H-[1,2,4]triazole-3-thiol (50 mg, 0.18 mmol) in dimethyl formamide (1.5 mL) was added N-(2-chloro-pyridin-3-yl)-2-chloro-acetamide (prepared from 2-Chloro-pyridin-3-ylamine) (37 mg, 0.18 mmol) and potassium carbonate (28 mg, 0.22 mmol) and the mixture stirred 18 h at room temperature. Water was added and the aqueous layer extracted with ethyl acetate. The organic layer was dried over sodium sulfate and concentrated. The resulting residue was purified by column chromatography (90% dichloromethane/10% methanol) to afford the desired product (40.1 mg, 52% yield).

### N-(2-Methyl-4-sulfamoyl-phenyl)-2-[5-methyl-4-(2,5,7-trimethyl-quinolin-8-yl)-4H-[1,2,4]triazol-3-ylsulfanyl]-acetamide

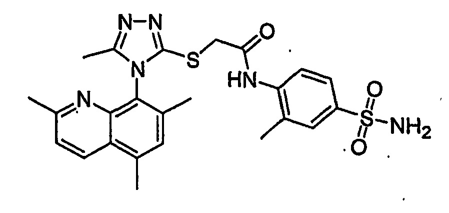

2,5,7-Trimethyl-quinoline: To a suspension of 3,5-dimethyl aniline (5 g, 41.2 mmol) in hydrochloric acid (20 mL) was added at 0 °C acetaldehyde drop-wise and the mixture stirred at this temperature for 15 min and then at 70 °C for 18 hours. The reaction mixture was then cooled to room temperature, poured into iced water, neutralized with ammonium hydroxide and extracted with ethyl acetate. The organic layer was dried over Na₂SO₄ and concentrated in vacuo. Column chromatography of the residue (hexane/ethyl acetate) (6:1) afforded (3.95g, 56% yield) of the desired quinoline.

2,5,7-Trimethyl-8-nitro-quinoline: Sulfuric acid (21.8 mL) was added to neat 2,5,7-trimethyl-quinoline (4 g, 23.4 mmol) at 0 °C. Potassium nitrate (2.6 g, 25.7 mmol) was then added in portions and the reaction stirred at 0 °C for 30 min and then at room temperature for 1 h. The reaction mixture was then poured over ice, neutralized with ammonium hydroxide and extracted with dichloromethane. The organic layer was dried over sodium sulfate and concentrated under reduced pressure. Column chromatography of the residue (hexane/ethyl acetate) (3:1) afforded 3.63 g, 72% yield of the desired quinoline.

2,5,7-Trimethyl-quinolin-8-ylamine: 2,5,7-Trimethyl-8-nitro-quinoline (3.63 g, 16.8 mmol) and sodium dithionite (14 g, 80.6 mmol) were heated under reflux in 50% aqueous ethanol (400 mL) for 4 hours. The mixture was made alkaline with 1M NaOH, and then extracted with ether. The combined organic layers were dried over sodium sulfate and concentrated under reduced pressure to give the desired compound (1.65 g, 54% yield).

5-Methyl-4-(2,5,7-trimethyl-quinolin-8-yl)-4H-[1,2,4]triazole-3-thiol: A solution ethyl acetimidate thiosemicarbazone (1.4 g, 8.8 mmol), dimethyl formamide (10 mL) and 2,5,7-trimethyl-quinolin-8-ylamine (1.64 g, 8.8 mmol) was heated at reflux for 3 hours. After evaporation of the solvent, a solution of 1 *N* sodium hydroxide was added (10 mL) and the mixture was stirred for 20 min at 40 °C. The reaction mixture was then extracted with ether to remove side products. The resulting aqueous layer was treated with a 10% solution of hydrochloric acid until the product precipitated (879 mg, 35% yield).

N-(2-Methyl-4-sulfamoyl-phenyl)-2-[5-methyl-4-(2,5,7-trimethyl-quinolin-8-yl)-4H-[1,2,4]triazol-3-ylsulfanyl]-acetamide: To a solution of 5-methyl-4-(2,5,7-trimethyl-quinolin-8-yl)-4H-[1,2,4]triazole-3-thiol (50 mg, 0.19 mmol) in dimethyl formamide (1.5 mL) was added N-(2-Methyl-4-sulfamoyl-phenyl)-2-chloro-acetamide (47 mg, 0.19 mmol) and potassium carbonate (28 mg, 0.21 mmol) and the mixture stirred 18 h at room temperature. Water was added and the aqueous layer extracted with ethyl acetate. The organic layer was dried over sodium sulfate and concentrated. The resulting residue was purified by column chromatography (90% dichloromethane/10% methanol) to afford the desired product (65 mg, 73% yield).

### N-(2-Chloro-pyridin-3-yl)-2-[4-(5-dimethylamino-2-methyl-quinolin-8-yl)-5-methyl-4H-[1,2,4]triazol-3-ylsulfanyl]-acetamide

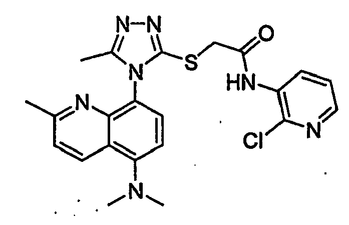

5-Chloro-2-methyl-8-nitro-quinoline: Hydrochloric acid (12 mL) was added at 0 °C to a flask containing 2-nitro-5-chloro aniline (3 g, 17.4 mmol), acetaldehyde (3 mL, 52.2 mmol) was then added drop-wise at 0 °C and the mixture stirred at this temperature for 15 min and then at 75 °C for 3 hours. The reaction mixture was then cooled to room temperature, poured into iced water, neutralized with ammonium hydroxide and extracted with ethyl acetate. The organic layer was dried over Na₂SO₄ and concentrated in vacuo to give 1.69 g, 44% yield of the nitro quinoline which was used in the next step without further purification.

Dimethyl-(2-methyl-8-nitro-quinolin-5-yl)-amine: To a solution of 5-chloro-2-methyl-8-nitro-quinoline (680 mg, 3.1 mmol) in dimehylformamide (3 mL) was added at room temperature dimethylamine (2M, MeOH) (16 mL, 31 mmol) and the reaction stirred for 4 days at 40 °C. The reaction mixture was then concentrated and purified by column chromatography (hexane/ethyl acetate) (3:1) to afford 557 mg, 78% yield of the desired amine.

2-Methyl-5-dimethylamino-8-arninoquinoline: Dimethyl-(2-methyl-8-nitro-quinolin-5-yl)-amine (556 mg, 2.4 mmol) and sodium dithionite (2.09 g, 12 mmol) were heated under reflux in 50% aqueous ethanol (60 mL) for 1 hours. The mixture was made alkaline with 1M NaOH, and then extracted with ether. The combined organic layers were dried over sodium sulfate and concentrated under reduced pressure to give the desired compound (450 mg, 94% yield).

4-(5-Dimethylamino-2-methyl-quinolin-8-yl)-5-methyl-4H-[1,2,4]triazole-3-thiol: A solution ethyl acetimidate thiosemicarbazone (361 mg, 2.2 mmol), dimethyl formamide (4 mL) and 2-methyl-5-dimethylamino-8-aminoquinoline (449 mg, 2.2 mmol) was heated at reflux for 3 hours. After evaporation of the solvent, a solution of 1 *N* sodium hydroxide was added (10 mL) and the mixture was stirred for 20 min at 40 °C. The reaction mixture was then extracted with ether to remove side products. The resulting aqueous layer was treated with a 10% solution of hydrochloric acid until the product precipitated (170 mg, 25% yield).

N-(2-Chloro-pyridin-3-yl)-2-[4-(5-dimethylamino-2-methyl-quinolin-8-yl)-5-methyl-4H-[1,2,4]triazol-3-ylsulfanyl]-acetamide: To a solution of 4-(5-dimethylamino-2-methyl-quinoliri-8-yl)-S-methyl-4H-[1,2,4]triazole-3-thiol (50 mg, 0.17 mmol) in dimethyl formamide (1.5 mL) was added N-(2-chloro-pyridin-3-yl)-2-chloro-acetamide (prepared from 2-Chloro-pyridin-3-ylamine) (35 mg, 0.17 mmol) and potassium carbonate (25 mg, 0.18 mmol) and the mixture stirred 18 h at room temperature. Water was added and the aqueous layer extracted with ethyl acetate. The organic layer was dried over sodium sulfate and concentrated. The resulting residue was purified by column chromatography (90% dichloromethane/10% methanol) to afford the desired product (63 mg, 79% yield).

### 2-[4-(4-Chloro-2-methoxy-5-methyl-quinolin-8-yl)-5-methyl-4H-[1,2,4]triazol-3-ylsulanyl]-N-(2-methyl-4-sulfamoyl-phenyl)-acetamide

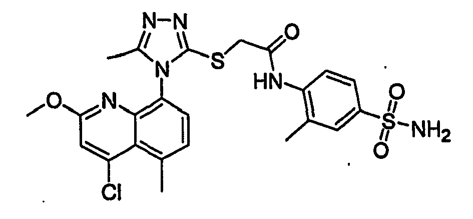

4-Chloro-2-methoxy-5-methyl-8-nitro-quinoline: 2,4-Dichloro-5-methylquinoline (1g, 3.9 mmol) was heated under reflux in sodium methoxide (0.5M, MeOH) (8 mL, 3.9 mmol) for 1 hour. The reaction was then cooled, poured into cold water and filtered to give 875 mg, 89% yield of the desired quinoline.

4-Chloro-2-methoxy-5-methyl-quinolin-8-ylamine: 4-Chloro-2-methoxy-5-methyl-8-nitro-quinoline (875 mg, 3.5 mmol) and sodium dithionite (3 g, 17.3 mmol) were heated under reflux in 50% aqueous ethanol (120 mL) for 1 hours. The mixture was made alkaline with 1M NaOH, and then extracted with ether. The combined organic layers, were dried over sodium sulfate and concentrated under reduced pressure to give the desired compound (410 mg, 53% yield).

4-(4-Chloro-2-methoxy-5-methyl-quinolin-8-yl)-5-methyl-4H-[1,2,4]triazole-3-thiol: A solution of ethyl acetimidate thiosemicarbazone (283 mg, 1.75 mmol), dimethyl formamide (4 mL) and 4-chloro-2-methoxy-5-methyl-quinolin-8-ylamine (2.83 mg, 1.75 mmol) was heated at reflux for 3 hours. After evaporation of the solvent, a solution of 1 *N* sodium hydroxide was added (10 mL) and the mixture was stirred for 20 min at 40 °C. The reaction mixture was then extracted with ether to remove side products. The resulting aqueous layer was treated with a 10% solution of hydrochloric acid until the desired product precipitated (170 mg, 25% yield).

2-[4-(4-Chloro-2-methoxy-5-methyl-quinolin-8-yl)-5-methyl-4H-[1,2,4]triazol-3-ylsulfanyl]-N-(2-methyl-4-sulfamoyl-phenyl)-acetamide: To a solution of 4-(4-chloro-2-methoxy-5-methyl-quinolin-8-yl)-5-methyl-4H-[1,2,4]triazole-3-thiol (50 mg, 0.16 mmol) in dimethyl formamide (1.5 mL) was added N-(2-Methyl-4-sulfamoyl-phenyl)-2-chloroacetamide (33 mg, 0.16 mmol) and potassium carbonate (25 mg, 0.18 mmol) and the mixture stirred 18 h at room temperature. Water was added and the aqueous layer extracted with ethyl acetate. The organic layer was dried over sodium sulfate and concentrated. The resulting residue was purified by column chromatography (90% dichloromethane/10% methanol) to afford the desired compound (42 mg, 49% yield).

### Triazole Derivatives 5-Methoxymethyl-4-phenyl-4H-[1,2,4]triazole-3-thiol

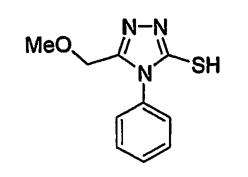

A 100 mL round-bottomed flask was charged with 4-phenylthiosemicarazide (500 mg, 2.99 mmol) in 20 mL absolute ethanol. The resulting heterogeneous reaction mixture was added ethyl methoxyacetate (214 µL, 2.99 mmol), and subsequently 0.5 M sodium methoxide in methanol (11.96 mL, 5.98 mmol). The reaction was refluxed for 36 h under argon atmosphere. The solvent was removed by a rotavapor after 1 mL acetic acid was added. The resulting solids were purified by flash column chromatography with a mixture of 5% methanol in dichloromethane. Grey-colored solids (64%, 361 mg) were obtained.

### N-(2-Chloro-phenyl)-2-(5-methoxymethyl-4phenyl-4H-[1,2,4]triazol-3-ylsulfanyl)-acetamide

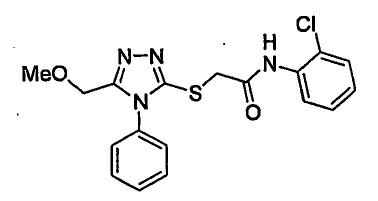

A 10 mL round-bottomed flask was charged with 5-methoxymethyl-4-phenyl-4H [1,2,4]triazole-3-tlriol (44.1 mg, 0.2 mmol), 2-Chloro-N-(2-chloro-phenyl)-acetamide (49.4 mg, 0.2 mmol), and potassium carbonate (30.4 mg, 0.22 mmol) in 2 mL dry DMF. The resulting heterogeneous solution was stirred at RT for 16 h. The solvent was removed by a vacuum-rotavapor yielding oily residue. The residue was purified by prep-TLC using 5% MeOH in dichloromethane yielding white solids (62 mg, 79%).

### (1-Methylsulfanyl-2-nitro-vinyl)-p-tolyl-amine

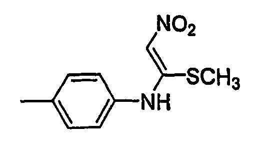

A 50 mL round-bottomed flask was charged with p-toluidine (1.65g, 10 mmol), and 1,1,-bis(methylthio)-2-nitroethene (1.07 g, 10 mmol) in 25 mL absolute EtOH. The reaction was refluxed for 5 h. Then, cooling down to room temperature yielded solids. These solids were washed with 20 mL EtOH (1.64 g, 73%). The solids were used for the next step without further purification.

### (1-Hydrazino-2-nitro-vinyl) p-tolyl-amine

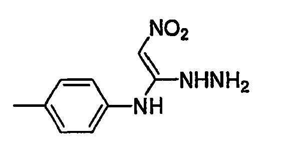

A 100 mL round-bottomed flask was charged with (1-methylsulfanyl-2-nitro-vinyl)-*p-*tolyl-amine (5.62 g, 25.08 mmol) in 60 mL EtOH. The resulting reaction mixture was added anhydrous hydrazine (1.88 mL, 60.2 mmol) by syringe at room temperature. The reaction was refluxed for 5 h. After cooling the mixture to room temperature, solids were precipitated from the solution. These solids were filtered by filter paper, then washed with 30 mL EtOH yielding yellow solids (4.30 g, 74 %).

### 3-Methyl-5-nitromethyl-4-p-tolyl-4H-[1,2,4]triazole

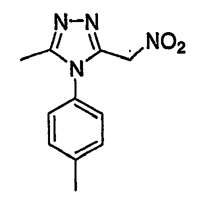

A 100 mL round-bottomed flask was charged with (1-hydxazino-2-nitro-vinyl)-*p-*tolyl-amine (4.3 g, 18.5 mmol) in 60 mL EtOH. The resulting reaction mixture was added triethyl orthoacetate (6.76 mL, 37.0 mmol). The reaction mixture was refluxed for 4 h. The solvent was removed by a rotavapor, and then the resulting residue was purified by flash-column chromatography with a mixture of n-hexanes and ethyl acetate (1:1). The very thick pure oily compound (3.7 g, 86%) was obtained.

### C-(5-Methyl-4-p-tolyl-4H-[1,2,4]triazol-3-yl)-methylamine

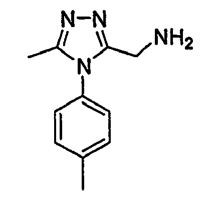

A 100 mL Parr-reaction vessel was charged with 3-methyl-5-nitro-4 *p-*tolyl-4H [1,2,4]triazole (350 mg, 1.43 mmol), 10% Pd-C (200 mg) in 40 mL MeOH. The reaction was shaken for 20 h at the pressure of 30 psi. The reaction was filtered over celite, and then the filtrate was concentrated under reduced pressure to pale yellow oil (280 mg, 97%). The residue was used for the next step without further purification.

### 1-(3-Bromo-phenyl)-3-(5-methyl-4-p-tolyl-4H-[1,2,4]triazol-3-ylmethyl)-urea

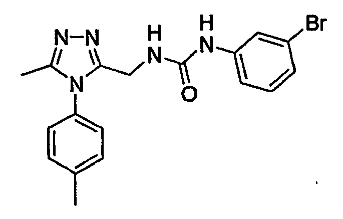

A 25 mL round-bottomed flask was charged with C-(5-Methyl-4-p-tolyl-4H-[1,2,4]triazol-3-yl)-methylamine (56 mg, 0.28 mmol) in dry methylene chloride (5 mL). The resulting solution was added 3-bromophenyl isocyanate (52 µL, 0.42 mmol). The reaction was stirred 15 h at room temperature. The solvent was removed by a rotavapor yielding brown residue. The residue was purified by prep-TLC with 5% MeOH in methylene chloride yielding 30 mg of white solids (27%).

### Imidazole derivatives 1-(4-Dimethylamino-naphthalen-1-yl)-3-prop-2-ynyl-thiourea

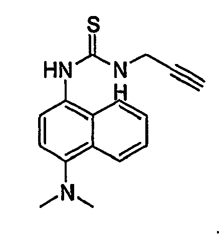

A 100 mL round-bottomed flask was charged with*p-*tolyl isothiocyanate (1.00 g, 6.70 mmol) in 20 mL benzene. The resulting solution was added propargylamine (0.51 mL, 7.37 mmol) by syringe. The reaction was stirred at room temperature for 2 h. The solvent was removed by a rotavapor, yielding light brown solids. The solids (1.29 g, 95%) were washed with 100 mL n-hexanes, and then the solids were used for the next step without further purification.

### 1-(4-Dimethylamino-naphthalen-1-yl)-5-methyl-1H-imidazole-2-thiol

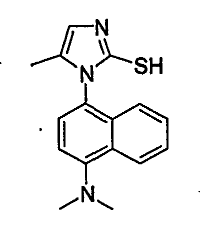

A 50 mL round bottomed-flask was charged with 1-(4-Dimethylamino-naphthalen-1-yl)-3-prop-2-ynyl-thiourea (160 mg, 0.56 mmol) in 10 mL MeOH. The resulting mixture was added 25% MeOH/MeONa (1mL) by a syringe under argon atmosphere. The reaction was stirred at room temperature for 5 h. The reaction mixture was added acetic acid (1 mL), and the solvent was removed by a rotavapor yielding white solids. The solids were purified by flash column chromatography with a mixture of hexanes and ethyl acetate (1:2) to yield 50 mg of pure white solids (31%).

### N-(2-Chloro-pyridin-3-yl)-2-[1-(4-dimethylamino-naphthalen-1-yl)-5-methyl-1H-imidazol-2-ylsulfanyl]-acetamide

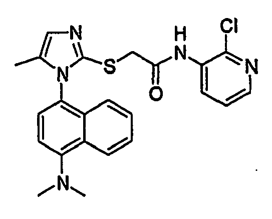

A 10 mL round-bottomed flask was charged with 1-(4-Dimethylamino-naphthalen-1-yl)-5-methyl-1H-imidazole-2-thiol (100 mg, 0.35 mmol), potassium carbonate (53.21 mg, 0.39 mmol) in 3 mL DMF. The resulting mixture was added 2-chloro-N-(2-chloro-pyridin-3-yl)-acetamide (72.05 mg, 0.35 mmol), and then the reaction was stirred for 16 h. The solvent was removed by a rotavapor yielding oily residue. The residue was purified by flash-column chromatography with a mixture of n-hexanes and ethyl acetate (1:1) to yield 136 mg of the pure product (86%).

### [1-(Methoxy-methyl-carbamoyl)-ethyl]-carbamic acid tert-butyl ester

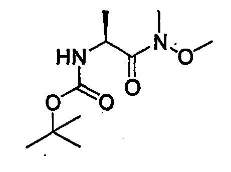

A 250 mL round-bottomed flask was charged with Boc-Ala-OH (1.89 g, 10 mmol) in 50 mL methylene chloride. The resulting mixture was added EDC (2.10 g, 11 mmol) was added in one portion. The reaction was stirred at room temperature for 10 min. The mixture was added the solution of a mixture of DIPEA (3.83 mL, 22 mmol) and N,O-dimethylhydroxylamine hydrochloride (1.07g, 11 mol) in 20 mL methylene chloride. The reaction was stirred for 14 h. The organic layer was washed with 50 mL, aq. HCl, and then 50 mL saturated aq. NaHCO₃ The organic layer was dried over anhydrous sodium sulfate. The solvent was removed by a rotavapor yielding yellow-red oil (5.3g, 53 %), which was used for the next step without further purification.

### (1-Methyl-2-oxo-propyl)-carbamic acid tert-butyl ester

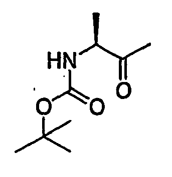

A 100 mL round-bottomed flask was charged with (1-methyl-2-oxo-propryl)-carbamic acid *tert*-butyl ester (1.23 g, 5.29 mmol) in 30 mL methylene chloride. The reaction mixture was added 3.0 M methylmagnesium bromide (5.30 mL, 15.9 mmol) by syringe. The reaction was stirred at room temperature for 30 min. The reaction was quenched with 100 mL 25 % aqueous ammonium chloride solution, diluting organic layer with 100 mL ethyl acetate. The combined organic layer was dried over anhydrous sodium sulfate, and then the solvent was removed by a rotavapor yielding yellow-brown oil (561 mg, 57 %). The crude product was used for the next step without further purification.

### 4,5-Dimethyl-1-p-tolyl-1H-imidazole-2-thiol

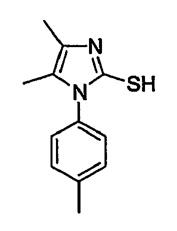

A 50 mL round-bottomed flask was charged with (1-Methyl-2-oxo-propyl)-carbamic acid *tert*-butyl ester (370 mg, 2.0 mmol) in 10 mL 25 % trifluoroacetic acid in methylene chloride. The resulting mixture was stirred at room temperature for 1 h. The solvent was removed by a rotavapor yielding dark-brown residue. Then the residue was redissolved in 20 mL methylene chloride. The resulting mixture was added a mixture of *p-*tolyl isothiocyanate and DIEA (2.8 mL, 8 mmol) in 10 mL methylene chloride. The resulting mixture was stirred at room temperature 18 h. The organic layer was washed with 50 mL saturated aqueous ammonium chloride solution. The organic layer was dried over anhydrous sodium sulfate yielding pale yellow residue. The residue was purified by flash silica-gel chromatography with a mixture of n-hexanes and ethyl acetate (1:1) to yield 40 mg of white solids (10 %).

*N-(2-Bromo-phenyl)-2-(4,5-dimethyl-1-p-tolyl-1H-imidazol-2-ylsulfanyl)-acetamide*

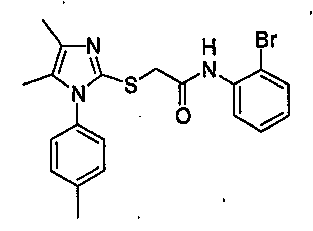

A 10 mL round-bottomed flask was charged with 4,5-dimethyl-1-*p*-tolyl-1*H-*imidazole-2-thiol (40 mg, 0.18 mmol), N-(2-Bromo-phenyl)-2-chloro-acetamide (45.3 mg, 018 mmol), and potassium carbonate (25 mg, 0.18 mmol) in 3 mL DMF. The reaction was stirred at room temperature 14 h. The solvent was removed under reduced pressure yielding yellow oily residue. The residue was purified by silica-gel chromatography with a mixture of ethyl acetate and n-hexanes (1:1) to yield 18 mg of white solids (23 %).

### Test System for Determination of Inhibition of HIV-1 Reverse Transcriptase

Contemplated compounds were screened for inhibitory activity against human immunodeficiency virus type 1 (HIV-1) using a high throughput cell-based assay using HIV-1 expressing firefly luciferase as a reporter gene and pseudotyped with vesicular stomatitis virus envelope glycoprotein (VSV-G). Experimental procedures were essentially as described by Connor et al. in Journal of Virology (1996), 70: 5306-5311 (Characterization of the functional properties of *env* genes from long-term survivors of human immunodeficiency virus type 1 infection), and Popik et al. in Journal of Virology (2002), 76: 4709-4722 (Human immunodeficiency virus type 1 uses lipid raft-colocalized CD4 and chemokine receptors for productive entry into CD4⁺ T cells). It should be particularly appreciated that the virus contains two introduced mutations in the RT gene (K103N and Y181C, created by PCR mutagenesis) that render the virus highly resistant to current non-nucleoside HIV-1 drugs. Virus stocks were generated by cotransfection of plasmid DNA encoding VSV-G with vector pNL4-3Env(-)Luc(+) into 293T cells. Sixty-four hours after transfection, virus-containing medium was collected by centrifugation and stored frozen at -80° C.

HeLa cells were infected with the VSV-G pseudotyped virus in the presence of screening compounds in a 384-well microtiter plate format. Forty-eight hours after initial infection, lysis buffer and Luciferase Assay Reagent (Promega) was added to the cells and luciferase activity was determined by counting the resultant luminescence using a LJL luminometer. Since the luciferase gene is carried in the virus genome, its expression level directly reflects the virus replication level in the presence of a compound.

To evaluate the activity of the compounds against wild type HIV-1, the HeLa-JC53 cell line that expresses high levels of CD4 and CCR5 (see *e.g.,* Platt et al. in Journal of Virology (1998), 72: 2855-2864: Effect of CCR5 and CD4 cell surface concentrations on infection by macrophagetropic isolates of human immunodeficiency virus type 1) was modified by isolation of a stable cell line that expresses luciferase under the control of the HIV-1 promoter (long terminal repeat, *i.e.*, LTR). HIV-1 infection of this cell line stimulates the transcription of luciferase from the HIV-1 promoter and the luciferase gene expression level is proportional to the level of virus replication (Harrington et al. in Journal of Virology Methods (2000), 88: 111-115: Direct detection of infection of HIV-1 in blood using a centrifugation-indicator cell assay; and Roos et al. in Virology (2000), 273: 307-315: LuSIV cells: a reporter cell line for the detection and quantitation of a single cycle of HIV and SIV replication). Procedures for virus infection, compound testing and luciferase activity determination were the same as for the VSV-G pseudotyped HIV-1.

Two approaches were used to evaluate the cytotoxicity of the positive compounds discovered in the HIV-1 virus assays. The first approach employed another modified HeLa-JC53 cell line that constitutively expresses high level of luciferase without virus infection. The level of luciferase expression in these cells served as an indicator for cell replication in the presence of the compounds. Procedures for compound testing and luciferase activity determination were the same as for the virus infection tests. The other toxicity assay utilized HeLe-JC53 cells and a commercially available MTS assay kit (Promega) that measures the mitochondria function of the cells.

### Results

**Table 1** below depicts values for inhibitory activity against HIV of exemplary compounds. Inhibitory activity is indicated as EC₅₀ in microM, and IC₅₀ is indicated in microM for wild-type HIV RT. Inhibitory activity at concentrations of less than 10 microM are labeled A, inhibitory activity at concentrations of between 10 microM to 100 microM are labeled B, and inhibitory activity at concentrations of greater than 100 microM are labeled C.

**Table 1**

| **ID** | **EC50** | **IC50** |
|---|---|---|
| 1 | A | A |
| 2 | A | A |
| 3 | B | A |
| 4 | C | A |
| 5 | C | A |
| 6 | B | A |
| 7 | B | A |
| 8 | B | A |
| 9 | B | A |
| 10 | C | N/D |
| 11 | C | N/D |
| 12 | C | N/D |
| 13 | C | N/D |
| 14 | C | N/D |
| 15 | C | N/D |
| 16 | C | N/D |
| 17 | C | N/D |
| 18 | C | N/D |
| 19 | C | N/D |
| 20 | C | N/D |
| 21 | C | N/D |
| 22 | C | N/D |
| 23 | C | N/D |
| 24 | C | N/D |
| 25 | C | N/D |
| 26 | B | N/D |
| 27 | C | N/D |
| 28 | C | N/D |
| 29 | C | B |
| 30 | C | N/D |
| 31 | C | N/D |
| 32 | C | N/D |
| 33 | C | B |
| 34 | C | B |
| 35 | C | N/D |
| 36 | C | N/D |
| 37 | C | N/D |
| 38 | N/D | B |
| 39 | C | N/D |
| 40 | C | B |
| 41 | C | B |
| 42 | C | N/D |
| 43 | C | B |
| 44 | C | B |
| 45 | C | 36.27 |
| 46 | C | C |
| 47 | C | N/D |
| 48 | C | N/D |
| 49 | C | N/D |
| 50 | C | N/D |
| 51 | C | N/D |
| 52 | C | B |

With respect to the particular compounds listed as Compound ID 1-52, **Table 2** below depicts the substituents for the respective compounds based on the scaffold as shown above Table 2. The structures of compounds with the ID 3, 10, 22, and 25 are depicted below Table 2.

**Table 2**

| | | | | | | | | |
|---|---|---|---|---|---|---|---|---|
| 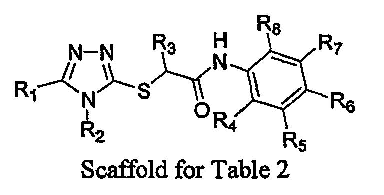 | | | | | | | | |

| ID | R₁ | R₂ | R₃ | R₄ | R₅ | R₆ | R₇ | R₈ |
|---|---|---|---|---|---|---|---|---|
| 1 | Me | 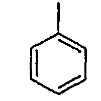 | H | Br | H | Me | H | H |
| 2 | Me | 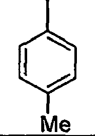 | H | Cl | H | H | H | H |
| 4 | 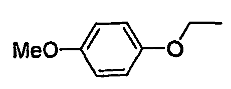 | 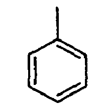 | H | Br | H | Me | H | H |
| 5 | 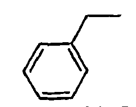 | 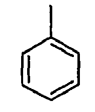 | H | Br | H | Me | H | H |
| 6 | Me | 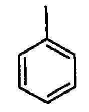 | H | Me | H | H | H | H |
| 7 | Me | 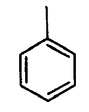 | H | Me | H | Me | H | H |
| 8 | 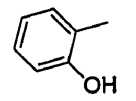 | 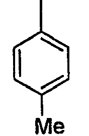 | H | Br | H | H | H | H |
| 9 | Me | 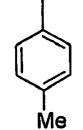 | H | Me | Me | H | H | H |
| 11 | 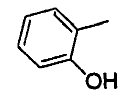 | H | H | H | H | F | H | H |
| 12 | 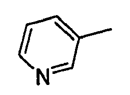 | Me | H | H | H | Me | H | H |
| 13 | Me | 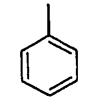 | H | H | H | NO₂ | H | H |
| 14 | Me | 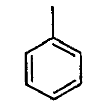 | H | Me | H | H | H | Me |
| 15 | Me | 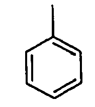 | H | H | H | F | H | H |
| 16 | Me | 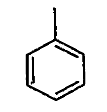 | Me | Me | H | H | H | H |
| 17 | 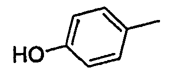 | Me | Me | H | H | Me | H | H |
| 18 | 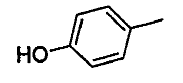 | Me | H | H | Me | H | Me | H |
| 19 | H | 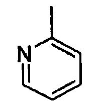 | H | Br | H | H | H | H |
| 20 | H | 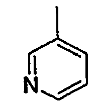 | H | CF₃ | H | H | H | H |
| 21 | H | 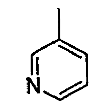 | H | H | CF₃ | Cl | H | H |
| 23 | Me | 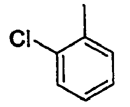 | H | CF₃ | H | H | H | H |
| 24 | 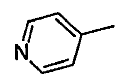 | 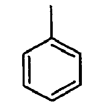 | H | I | H | H | H | H |
| 26 | 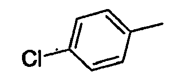 | H | H | Me | H | H | H | H |
| 27 | 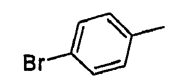 | Me | H | H | H | H | H | H |
| 28 | 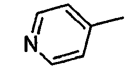 | 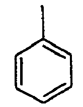 | H | Br | H | Me | H | H |
| 29 | 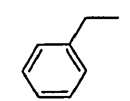 | Me | H | Br | H | Me | H | H |
| 30 | Me | 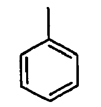 | H | H | Me | Me | H | H |
| 31 | 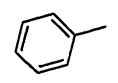 | Me | H | Br | H | Me | H | H |
| 32 | 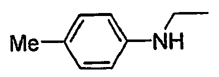 | Me | H | Br | H | Me | H | H |
| 33 | 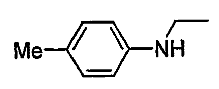 | 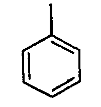 | H | Br | H | Me | H | H |
| 34 | 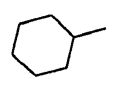 | 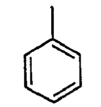 | H | Br | H | H | H | H |
| 35 | 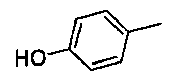 | 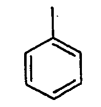 | H | Br | H | H | H | H |
| 36 | 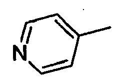 | 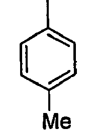 | H | Br | H | Me | H | H |
| 37 | 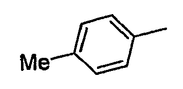 | Me | H | Br | H | H | H | H |
| 38 | 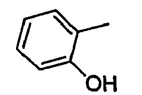 | 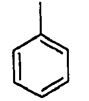 | H | Br | H | H | H | H |
| 39 | Me | 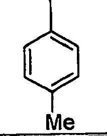 | H | H | Cl | Me | H | H |
| 40 | Me | 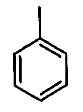 | H | H | Me | H | H | H |
| 41 | Me | 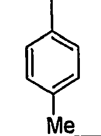 | H | H | H | C(O)Me | H | H |
| 42 | 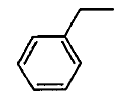 | 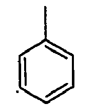 | H | H | H | Me | H | H |
| 43 | 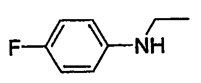 | 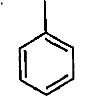 | H | Br | H | Me | H | H |
| 44 | 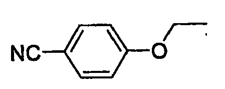 | 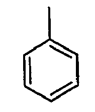 | H | Br | H | Me | H | H |
| 45 | Me | 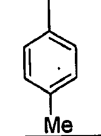 | Me | H | H | Me | H | H |
| 46 | 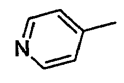 | 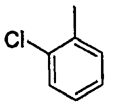 | H | Br | H | Me | H | H |
| 47 | Me | 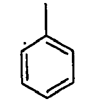 | H | H | H | Me | H | H |
| 48 | 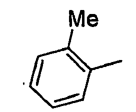 | Me | H | Br | H | Me | H | H |
| 49 | Me | 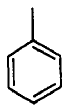 | H | H | H | H | H | H |
| 50 | 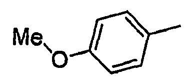 | 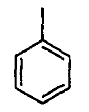 | H | Br | H | H | H | H |
| 51 | Me | 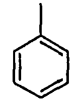 | H | Me | H | Me | H | Me |
| 52 | 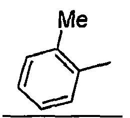 | 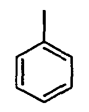 | H | Br | H | Me | H | H |

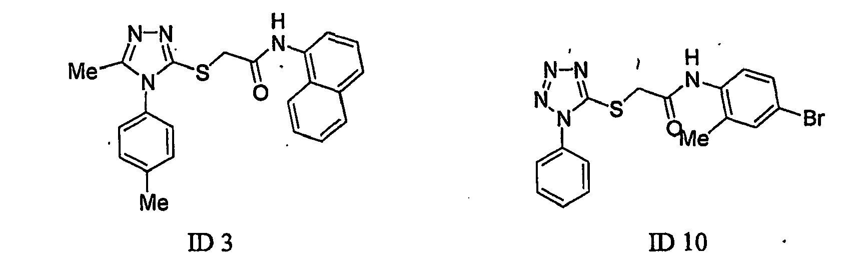

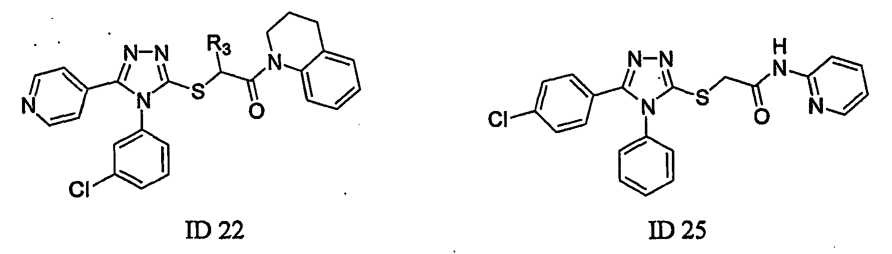

**Table 3** below depicts values for inhibitory activity against HIV of exemplary compounds. Inhibitory activity is indicated as EC₅₀ in microM, and IC₅₀ is indicated in microM for wild-type HIV RT. Inhibitory activity at concentrations of less than 10 microM are labeled A, inhibitory activity at concentrations of between 10 microM to 100 microM are labeled B, and inhibitory activity at concentrations of greater than 100 microM are labeled C.

**Table 3**

| **ID** | **EC50** | **IC50** |
|---|---|---|
| 1 | A | A |
| 2 | A | A |
| 3 | A | A |
| 4 | A | A |
| 5 | A | A |
| 6 | A | A |
| 7 | A | A |
| 8 | A | A |
| 9 | A | A |
| 10 | A | A |
| 11 | A | A |
| 12 | A | A |
| 13 | A | A |
| 14 | A | A |
| 15 | A | A |
| 16 | A | A |
| 17 | A | A |
| 18 | A | A |
| 19 | A | A |
| 20 | A | A |
| 21 | A | A |

With respect to the particular compounds listed as Compound ID 1-21, **Table 4** below depicts the substituents for the respective compounds based on the scaffold as shown above Table 4. The structures of compounds with the ID 19, 20, and 21 are depicted below Table 4.

**Table 4**

| | | | | | | | | |
|---|---|---|---|---|---|---|---|---|
| 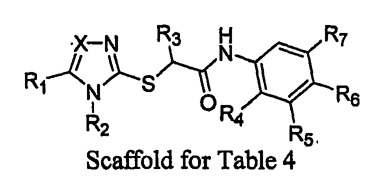 | | | | | | | | |

| ID | R₁ | R₂ | R₃ | R₄ | R₅ | R₆ | R₇ | X |
|---|---|---|---|---|---|---|---|---|
| 1 | Me | 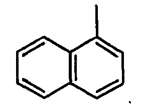 | H | Cl | H | H | | H N |
| 2 | Me | 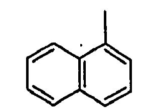 | H | Cl | H | H | H | CH |
| 4 | Me | 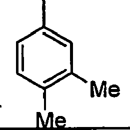 | H | Me | H | S(O)₂NH₂ | H | N |
| 5 | Me | 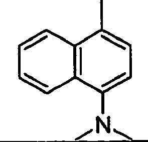 | Me | Cl | H | S(O)₂NH₂ | H | CH |
| 6 | Me | 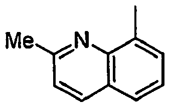 | H | Me | H | S(O)₂NH₂ | H | N |
| 7 | Me | 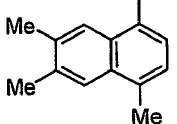 | H | Br | H | H | H | N |
| 8 | Me | 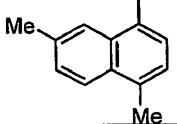 | H | Cl | H | S(O)₂NH₂ | H | N |
| 9 | Me | 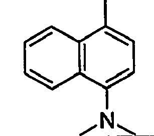 | H | Me | H | S(O)₂NH₂ | H | N |
| 11 | Me | 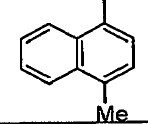 | H | CF₃ | H | H | H | N |
| 12 | CF₃ | 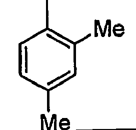 | H | Me | H | S(O)₂NH₂ | H | N |
| 13 | Me | 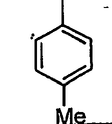 | H | C(O)Me | H | H | H | N |
| 14 | Me | 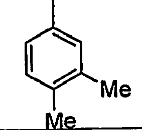 | H | Cl | H | C(O)OMe | H | N |

**Table 4**

| | | | | | | | | |
|---|---|---|---|---|---|---|---|---|
| 15 | Me | 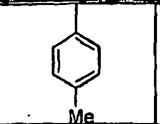 | H | SMe | H | H | H | N |
| 16 | Me | 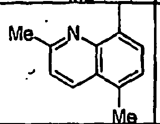 | H | Cl | H | S(O)₂NH₂ | H | N |
| 17 | Br | 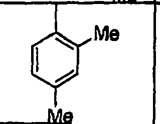 | H | Me | H | S(O)₂NH₂ | H | N |
| 18 | Et | 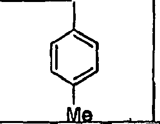 | H | Br | H | H | H | N |

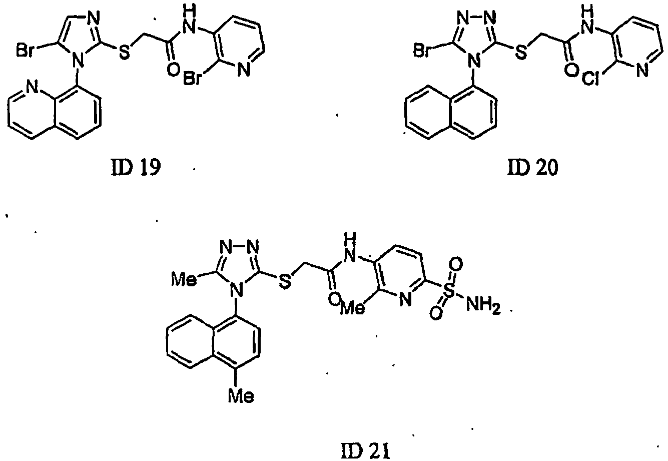

Thus, specific embodiments and applications of non-nucleoside reverse transcriptase inhibitors have been disclosed.

## Claims

1. A compound having a structure according to Formula
HET-W-C(R₁)(R₂)-C(Y)-N(R₄R₅)
wherein HET is a disubstituted 1,2,4-triazole or a disubstituted imidazole, wherein one substituent of HET is a substituted aryl, wherein each substituent of the aryl is independently hydroxyl, thiol, alkylthiol, halogen, alkoxy, amino, amido, nitro, carboxyl, cycloalkyl of 3 to 15 carbon atoms, heterocycle, cycloheteroalkyl, acyl, aryl, aryloxy, heteroaryl, arylalkyl, heteroarylalakyl, alkyl, alkenyl, alkynyl, and cyano, wherein the substituted aryl is covalently bound to a nitrogen of HET, and wherein the other substituent of HET is methyl, halogen or trifluoromethyl;
W is O, S, S(O), S(O)₂, NH, NR₁ or CH₂;
R₁ and R₂ are independently hydrogen, methyl, ethyl, n-propyl, i-propyl, n-butyl, t-butyl, i-butyl, i-amyl, n-amyl, hexyl, alkenyl of 1 to 10 carbon atoms, alkynyl of 1 to 10 carbon atoms, halogen, OH, SH, NH₂, N₃, O-methyl, O-ethyl, O-n-propyl, O-i-propyl, O-n-butyl, O-t-butyl, O-i-butyl, O-i-amyl, O-n-amyl, O-hexyl, or CH₂OH; Y is O, S, or NR₃, wherein R₃ is hydrogen, methyl, ethyl, n-propyl, i-propyl, n-butyl, t-butyl, i-butyl, i-amyl, n-amyl or hexyl, alkenyl of 1 to 10 carbon atoms, alkynyl of 1 to 10 carbon atoms, or hydroxy, O-alkyl, or CH₂OH;
R₄ is hydrogen, methyl, ethyl, n-propyl, i-propyl, n-butyl, t-butyl, i-butyl, i-amyl, n-amyl, hexyl, alkenyl of 1 to 10 carbon atoms, or alkynyl of 1 to 10 carbon atoms; and R₅ is an optionally substituted aryl, wherein each substituent is independently a hydroxyl, a thiol, a halogen, an amino, an amido, a nitro, a carboxyl, a cycloalkyl of 3 to 15 carbon atoms, a cyano, a methyl, an ethyl, an n-propyl, an i-propyl, a pyridinyl, a morpholino, a furyl, a quinolino, a naphthyl, a methoxy, an ethoxy, an n-propyloxy, an i-propyloxy, an n-butyloxy, an i-butyloxy, a t-butyloxy, a phenyloxy or a naphthyloxy group.

2. The compound of claim 1, wherein R₁, R₂ and R₄ are hydrogen, and wherein one substituent of HET is methyl, ethyl, n-propyl, i-propyl, n-butyl, t-butyl, i-butyl, i-amyl, n-amyl, hexyl or halogen.

3. The compound of claim 2, wherein Y is O.

4. The compound of claim 1, wherein R₄ is hydrogen and R₅ is a substituted aryl.

5. The compound of claim 1 or claim 4, wherein the substituted aryl covalently bound to a nitrogen of HET is a substituted naphthyl, wherein the substituent is a hydroxyl, a thiol, a halogen, an amino, an amido, a nitro, a carboxyl, a cycloalkyl of 3 to 15 carbon atoms, a cyano, a methyl, an ethyl, an n-propyl, an i-propyl, a pyridinyl, a morpholino, a furyl, a quinolino, a naphthyl, a methoxy, an ethoxy, an n-propyloxy, an i-propyloxy, an n-butyloxy, an i-butyloxy, a t-butyloxy, a phenyloxy or a naphthyloxy group.

6. The compound of claims 4 or 5, wherein R₅ is an ortho-substituted phenyl in which the ortho-substituent is a halogen or methyl.

7. The compound of claim 6, wherein R₅ further comprises a para-substituent.

8. The compound of any of claims 4 to 7, wherein R₅ is an ortho-substituted phenyl that further comprises a para-substituent.

9. A compound according to any of claims 1 to 8 for use in the treatment of HIV infection.

## Patentansprüche

1. Verbindung mit einer Struktur gemäß der Formel
HET-W-C(R₁)(R₂)-C(Y)-N(R₄R₅)
wobei HET ein disubstituiertes 1,2,4-Triazol oder ein disubstituiertes Imidazol ist, wobei ein Substituent von HET substituiertes Aryl ist, wobei jeder Substituent des Aryls unabhängig voneinander Hydroxyl, Thiol, Alkylthiol, Halogen, Alkoxy, Amino, Amido, Nitro, Carboxyl, Cycloalkyl mit 3 bis 15 Kohlenstoffatomen, Heterocyclen, Cycloheteroalkyl, Acyl, Aryl, Aryloxy, Hetereoaryl, Arylalkyl, Heteroarylalkyl, Alkyl, Alkenyl, Alkinyl und Cyano ist, wobei das substituierte Aryl kovalent mit einem Stickstoff des HET verbunden ist, und wobei der andere Substituent des HET Methyl, Halogen oder Trifluoromethyl ist;
W O, S, S(O), S(O)₂, NH, NR₁ oder CH₂ ist;
R₁ und R₂ unabhängig voneinander Wasserstoff, Methyl, Ethyl, n-Propyl, i-Propyl, n-Butyl, t-Butyl, i-Butyl, i-Amyl, n-Amyl, Hexyl, Alkenyl mit 1 bis 10 Kohlenstoffatomen, Alkinyl mit 1 bis 10 Kohlenstoffatomen, Halogen, OH, SH, NH₂, N₃, O-Methyl, O-Ethyl, O-n-Propyl, O-i-Propyl, O-n-Butyl, O-t-Butyl, O-i-Amyl, O-n-Amyl, O-Hexyl oder CH₂OH sind;
Y O, S oder NR₃ ist, wobei R₃ Wasserstoff, Methyl, Ethyl, n-Propyl, i-Propyl, n-Butyl, t-Butyl, i-Butyl, i-Amyl, n-Amyl oder Hexyl, Alkenyl mit 1 bis 10 Kohlenstoffatomen, Alkinyl mit 1 bis 10 Kohlenstoffatomen oder Hydroxy, O-Alkyl oder CH₂OH ist;
R₄ Wasserstoff, Methyl, Ethyl, n-Propyl, i-Propyl, n-Butyl, t-Butyl, i-Butyl, i-Amyl, n-Amyl, Hexyl, Alkenyl mit 1 bis 10 Kohlenstoffatomen oder Alkynyl mit 1 bis 10 Kohlenstoffatomen ist; und R₅ ein optional substituiertes Aryl ist, wobei jeder Substituent unabhängig voneinander eine Hydroxyl, eine Thiol, eine Halogen, eine Amino, eine Amido, eine Nitro, eine Carboxyl, eine Cycloalkyl mit 3 bis 15 Kohlenstoffatomen, eine Cyano, eine Methyl, eine Ethyl, eine n-Propyl, eine i-Propyl, eine Pyridinyl, eine Morpholino, eine Furyl, eine Chinolino, eine Naphthyl, eine Methoxy, eine Ethoxy, eine n-Propyloxy, eine i-Propyloxy, eine n-Butyloxy, eine i-Butyloxy, eine t-Butyloxy, eine Phenyloxy oder eine Naphthyloxy Gruppe ist.

2. Die Verbindung nach Anspruch 1, wobei R₁, R₂ und R₄ Wasserstoff sind und wobei ein Substituent von HET Methyl, Ethyl, n-Propyl, i-Propyl, n-Butyl, t-Butyl, i-Butyl, i-Amyl, n-Amyl, Hexyl oder Halogen ist.

3. Die Verbindung nach Anspruch 2, wobei Y O ist.

4. Die Verbindung nach Anspruch 1, wobei R₄ Wasserstoff und R₅ substituiertes Aryl ist.

5. Die Verbindung nach Anspruch 1 oder Anspruch 4, wobei das substituierte Aryl, das kovalent mit einem Sticksoff des HET verbunden ist, substituiertes Naphthyl ist, wobei der Substituent eine Hydroxyl, eine Thiol, eine Halogen, eine Amino, eine Amido, eine Nitro, eine Carboxyl, eine Cycloalkyl mit 3 bis 15 Kohlenstoffatomen, eine Cyano, eine Methyl, eine Ethyl, eine n-Propyl, eine i-Propyl, eine Pyridinyl, eine Morpholino, eine Furyl, eine Chinolino, eine Naphthyl, eine Methoxy, eine Ethoxy, eine n-Propyloxy, eine i-Propyloxy, eine n-Butyloxy, eine i-Butyloxy, eine t-Butyloxy, eine Phenyloxy oder eine Naphthyloxy Gruppe ist.

6. Die Verbindung nach Anspruch 4 oder 5, wobei R₅ ein ortho-substiuiertes Phenyl ist, wobei der Ortho-Substituent ein Halogen oder Methyl ist.

7. Die Verbindung nach Anspruch 6, wobei R₅ zusätzlich einen Para-Substituenten beinhaltet.

8. Die Verbindung nach einem der Ansprüche 4 bis 7, wobei R₅ ein ortho-substituiertes Phenyl ist, das zusätzlich einen Para-Substituenten beinhaltet.

9. Die Verbindung nach einem der Ansprüche 1 bis 8 zur Verwendung bei der Behandlung einer HIV-Infektion.

## Revendications

1. Composé ayant une structure selon la formule
HET-W-C(R₁)(R₂)-C(Y)-N(R₄R₅)
dans laquelle HET est un 1,2,4-triazole disubstitué ou un imidazole disubstitué, où un substituant de HET est un aryle substitué, où chaque substituant de l'aryle est indépendamment un hydroxyle, un thiol, un alkylthiol, un halogène, un alcoxy, un amino, un amido, un nitro, un carboxyle, un cycloalkyle de 3 à 15 atomes de carbone, un hétérocycle, un cyclohétéroalkyle, un acyle, un aryle, un aryloxy, un hétéroaryle, un arylalkyle, un hétéroarylalakyle, un alkyle, un alcényle, un alcynyle, et un cyano, où l'aryle substitué est lié de manière covalente à un azote de HET, et où l'autre substituant de HET est un méthyle, un halogène ou un trifluorométhyle ;
W est O, S, S(O), S(O)₂, NH, NR₁ ou CH₂ ;
R₁ et R₂ représentent indépendamment un hydrogène, un méthyle, un éthyle, un n-propyle, un i-propyle, un n-butyle, un t-butyle, un i-butyle, un i-amyle, un n-amyle, un hexyle, un alcényle de 1 à 10 atomes de carbone, un alcynyle de 1 à 10 atomes de carbone, un halogène, OH, SH, NH₂, N₃, un O-méthyle, un O-éthyle, un O-n-propyle, un O-i-propyle, un O-n-butyle, un O-t-butyle, un O-i-butyle, un O-i-amyle, un O-n-amyle, un O-hexyle, ou CH₂OH;
Y est O, S, ou NR₃, où R₃ est un hydrogène, un méthyle, un éthyle, un n-propyle, un i-propyle, un n-butyle, un t-butyle, un i-butyle, un i-amyle, un n-amyle ou un hexyle, un alcényle de 1 à 10 atomes de carbone, un alcynyle de 1 à 10 atomes de carbone, ou un hydroxy, un O-alkyle, ou CH₂OH ;
R₄ est un hydrogène, un méthyle, un éthyle, un n-propyle, un i-propyle, un n-butyle, un t-butyle, un i-butyle, un i-amyle, un n-amyle, un hexyle, un alcényle de 1 à 10 atomes de carbone, ou un alcynyle de 1 à 10 atomes de carbone ;
et R₅ est éventuellement un aryle substitué, où chaque substituant est indépendamment un groupe hydroxyle, un groupe thiol, un groupe halogène, un groupe amino, un groupe amido, un groupe nitro, un groupe carboxyle, un groupe cycloalkyle de 3 à 15 atomes de carbone, un groupe cyano, un groupe méthyle, un groupe éthyle, un groupe n-propyle, un groupe i-propyle, un groupe pyridinyle, un groupe morpholino, un groupe furyle, un groupe quinolino, un groupe naphthyle, un groupe méthoxy, un groupe éthoxy, un groupe n-propyloxy, un groupe i-propyloxy, un groupe n-butyloxy, un groupe i-butyloxy, un groupe t-butyloxy, un groupe phényloxy ou un groupe naphthyloxy.

2. Composé selon la revendication 1, dans lequel R₁, R₂ et R₄ représentent un hydrogène, et où un substituant de HET est un méthyle, un éthyle, un n-propyle, un i-propyle, un n-butyle, un t-butyle, un i-butyle, un i-amyle, un n-amyle, un hexyle ou un halogène.

3. Composé selon la revendication 2, dans lequel Y est O.

4. Composé selon la revendication 1, dans lequel R₄ est un hydrogène et R₅ un aryle substitué.

5. Composé selon la revendication 1 ou 4, dans lequel l'aryle substitué lié de manière covalente à un azote de HET est un naphthyle substitué, le substituant étant un groupe hydroxyle, un groupe thiol, un groupe halogène, un groupe amino, un groupe amido, un groupe nitro, un groupe carboxyle, un groupe cycloalkyle de 3 à 15 atomes de carbone, un groupe cyano, un groupe méthyle, un groupe éthyle, un groupe n-propyle, un groupe i-propyle, un groupe pyridinyle, un groupe morpholino, un groupe furyle, un groupe quinolino, un groupe naphthyle, un groupe méthoxy, un groupe éthoxy, un groupe n-propyloxy, un groupe i-propyloxy, un groupe n-butyloxy, un groupe i-butyloxy, un groupe t-butyloxy, un groupe phényloxy ou un groupe naphthyloxy.

6. Composé selon les revendications 4 ou 5, dans lequel R₅ est un phényle substitué en ortho, où le substituant en ortho est un halogène ou un méthyle.

7. Composé selon la revendication 6, dans lequel R₅ comprend en outre un substituant en para.

8. Composé selon l'une quelconque des revendications 4 à 7, dans lequel R₅ est un phényle substitué en ortho qui comprend en outre un substituant en para.

9. Composé selon l'une quelconque des revendications 1 à 8, destiné à être utilisé dans le traitement d'une infection par le VIH.
